# EUROPEAN PATENT APPLICATION

(11) **EP 4 623 928 A1**
(43) Date of publication of application: **01.10.2025**
(21) Application number: 24167869.7
(22) Date of filing: 29.03.2024
(51) Int. Cl.: A61K 38/22, A61K 31/70, A61K 38/26, A61K 39/395, A61K 48/00, A61P 35/00

(54) **GLP-1R ANTAGONISTS FOR THE TREATMENT OF CANCER**

(71) Applicant: Fiorina, Paolo, 20054 Milano Due, Segrate (MI) (IT); Ben Nasr, Moufida, 20054 Milano Due, Segrate (MI) (IT)
(72) Inventor: Fiorina, Paolo, 20054 Milano Due, Segrate (MI) (IT); Ben Nasr, Moufida, 20054 Milano Due, Segrate (MI) (IT)
(74) Representative: Currado, Luisa

(57) **Abstract**

The present invention relates to GLP-1R antagonists or inhibitors, or pharmaceutical composition comprising thereof, for use in the treatment and/or prevention of a cancer.

## Description

### TECHNICAL FIELD

The present invention relates to GLP-1R antagonists for use in the prevention and/or treatment of cancer.

### BACKGROUND ART

Glucagon Like Peptide-1 (GLP-1) regulates glucose metabolism by signaling through its receptor (GLP-1R), which is expressed by pancreatic β-cells, gastric mucosa and the hypothalamic appetite center and skeletal muscle (1-8). GLP-1R agonists reduce the incidence and severity of severe diabetes complications, including cardiovascular events and diabetic kidney disease (9). Beside these well known metabolic effects, an increasing body of literature suggests that GLP-1R may have a role in regulating the immune system (10-12). GLP-1R mRNA expression has been detected in various populations of immune cells, including dendritic cells and T lymphocytes; authors have suggested that its signaling may have immunological properties (11). More recently, GLP-1R expression was confirmed in a subset of gut intra-epithelial T lymphocytes (13). These cells control the systemic metabolism by modulating hormone incretion from the entero-endocrine epithelial cells. In patients affected simultaneously by diabetes and psoriasis, evidences of an improvement of dermatological symptoms after the treatment with the GLP-1R agonist Exendin-4 were reported (14). Of note, after some patients discontinued the drug as consequence of mild adverse events, psoriasis symptoms substantially worsened but improved again when Exendin-4 was restarted. In non-obese diabetic (NOD) mice, a strain prone to autoimmune diabetes, Exendin-4 treatment delayed diabetes onset as well as islet allograft rejection in a fully mismatch model of islet allotransplantation (15, 16). More recently, the administration of GLP-1R agonist, Liraglutide, was shown to mitigate nephrotoxic serum nephritis in mice by dampening renal infiltration and the proliferation of effector T cells (17).

However, there is no conclusive evidence about a possible immunological role of GLP-1R and its signaling.

Despite the success of immune-checkpoint inhibitors therapies for cancer, failure of some immunotherapies upon their clinical translation posits for implementing/redesigning new effective immunotherapeutic targets (18, 19). The main aspects/determinants of the immune-oncology therapeutic failures reside mainly within the complexity of the immune cell-cancer environment (18). Indeed, future therapeutics as evidenced by several data emerging from tumor biology must focus on main 2 key factors amongst others, mainly related to T cells: rescuing T cell exhaustion (dysfunction) and homing in the tumor microenvironment (20).

Therefore, there is still the need of a treatment for cancer, in particular of an immunotherapy treatment which might be alternative or supportive of current immunotherapy treatments.

### SUMMARY OF THE INVENTION

It has now been found that GLP-1R antagonists can be advantageously used as immune check point inhibitors to stimulate anti-tumor immunity. In particular, the inventors found that GLP-1R antagonism with Exendin fragment (9-39) applied into a relevant murine cancer model *in vivo* was able to trigger an anti-tumor immune response.

Therefore, it is an object of the present invention a GLP-1R antagonist or inhibitor for use for the prevention and/or treatment of a cancer.

Preferably, said GLP-1R antagonist is selected from the group consisting of Exendin fragment (9-39), Exendin-3 (9-39) amide, GLP-1R Antagonist 1 (compound 5d), TB-001-003, TB-222-023 or Avexitide, VU 0650991, GLP-1 (9-36) amide and a monoclonal antibody anti-GLP-1R.

Preferably said tumor is a solid tumor selected from the group consisting of colorectal cancer, gastrointestinal cancer, pancreatic cancer, lung cancer, breast cancer, bladder cancer, prostate cancer, testicular cancer, ovarian cancer, uterine cancer, stomach cancer, liver cancer, renal cancer, melanoma, retinoblastoma, neuroblastoma, and sarcoma, or a liquid tumor selected from the group consisting of leukemias, lymphoma, and multiple myeloma, preferably it is a gastrointestinal cancer.

In a preferred embodiment, the GLP-1R antagonist or inhibitor is for use for the prevention and/or treatment of a cancer in a subject who is or has been treated with an immune checkpoint inhibitor, such as antibodies anti-PD-1, anti-PDL-1 or anti-CTLA-4. A pharmaceutical composition comprising at least one GLP-1R antagonist or inhibitor and at least one pharmaceutically acceptable carrier, excipient or stabilizer for use for the prevention and/or treatment of a cancer is also an object of the invention. Said pharmaceutical composition may also further comprise a further anti-cancer therapeutic agent.

### DETAILED DESCRIPTION OF THE INVENTION

### Figures

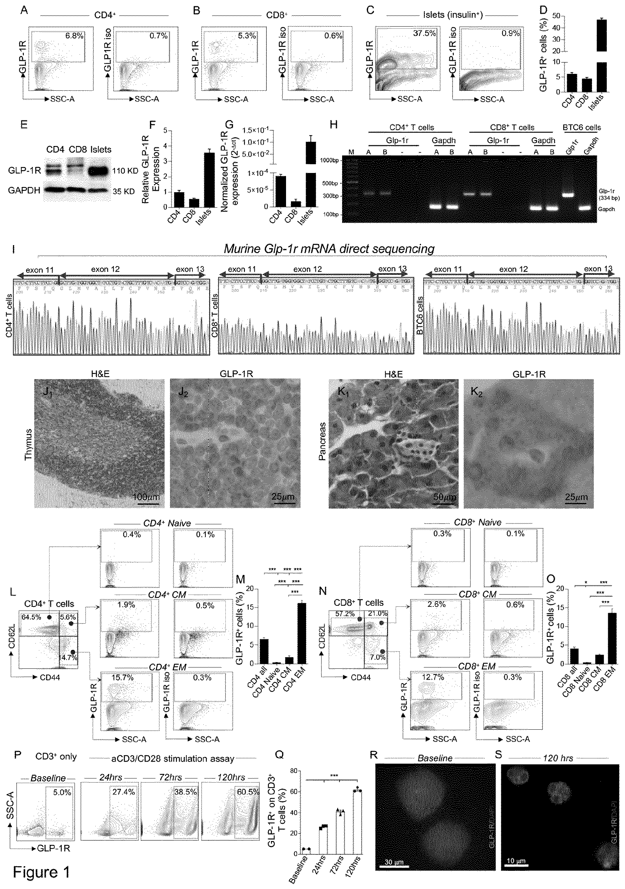

**Figure 1****. GLP-1R is expressed in murine T cells and it expands during TCR stimulation. A-B:** Representative dot plot of GLP-1R expression by murine splenic CD4⁺ and CD8⁺ cells and related isotype controls. **C:** Representative dot plots of GLP-1R expression by murine pancreatic β-cells and associated isotype controls. **D:** Bar graph depicts results of flow cytometric analysis of GLP-1R expression by CD4⁺ and CD8⁺ T lymphocytes, together with pancreatic β-cells as positive control. **E-F:** Western blot analysis and associated quantification confirmed GLP-1R expression on CD4⁺ and CD8⁺ T lymphocytes; pancreatic β-cells represent the positive control. **G:** Bar graph representing normalized mRNA expression of *Glp-1r* in CD4⁺ and CD8⁺ T lymphocytes; pancreatic β-cells represent the positive control. **H:** *Glp-1r* mRNA expression was assessed by RT-qPCR in 2 biological replicates of respectively CD4⁺, CD8⁺ T cells and in BTC6 cell line (used as positive control); reactions in which reverse transcriptase was omitted (-RT) were performed in parallel to test for DNA contamination; agarose gel electrophoresis analysis confirmed that the apparent size of the obtained qRT-PCR products was compatible to the expected amplicon length of 334 bp. **I:** Sanger sequencing further confirmed the identity of the amplification products and the expression of *Glp-1r* mRNA in CD4⁺ and CD8⁺ T cells. **J₁-J₂**: Representative images of hematoxylin and eosin (H&E) histology staining and immunoreactive GLP-1R^{pos} cells in murine thymus. **K₁-K₂:** Hematoxylin and eosin (H&E) histology staining, and immunoreactive GLP-1R^{pos} cells in murine pancreas. **L:** Representative dot plots for gating strategy and GLP-1R expression by CD4⁺CD44⁻CD62L⁺ naive T cells, CD4⁺CD44⁺CD62L⁺ memory T cells and CD4⁺CD44⁺CD62L⁻ effector T cells. **M:** Quantification of flow cytometric analysis from J and comparison with whole CD4⁺ population. **N:** Representative dot plots for gating strategy and GLP-1R expression by CD8⁺CD44⁻CD62L⁺ naive T cells, CD8⁺CD44⁺CD62L⁺ memory T cells and CD8⁺ CD44⁺CD62L⁻ effector T cells. **O:** Quantification of flow cytometric analysis from L and comparison with whole CD8⁺ population. **P-Q:** Cell trace violet-labelled CD3⁺ T cells were activated with anti-CD3/CD28 Abs. After 24 hours, 72 hours and 120 hours cells were harvested and stained for GLP-1R; representative dot plots for FACS analysis related to each time point (24, 72 and 120 hours) are shown in (P) and their corresponding bargraphs are shown in **(Q). R-S:** Immunofluorescence staining depicting GLP-1R expression on murine CD3⁺ T cells at baseline as shown in (R) and on murine CD3⁺ T cells stimulated for 120 hours with soluble anti-CD3/CD28 Abs (S). Data are representative of at least n=2 samples and are expressed as mean ± standard error of the mean (SEM). **P*<0.05; ***P*<0.01; ****P*<0.001. **Abbreviations:** GLP-1R, Glucagon-like peptide 1 receptor; BTC6, Beta-TC-6 *cell* line; qRT-PCR, Two-step quantitative reverse transcriptase-PCR; -RT, minus reverse transcriptase; H&E, hematoxylin and eosin; FACS, fluorescence-activated cell sorter scan; Abs, antibodies; EM, effector memory; CM, central memory.

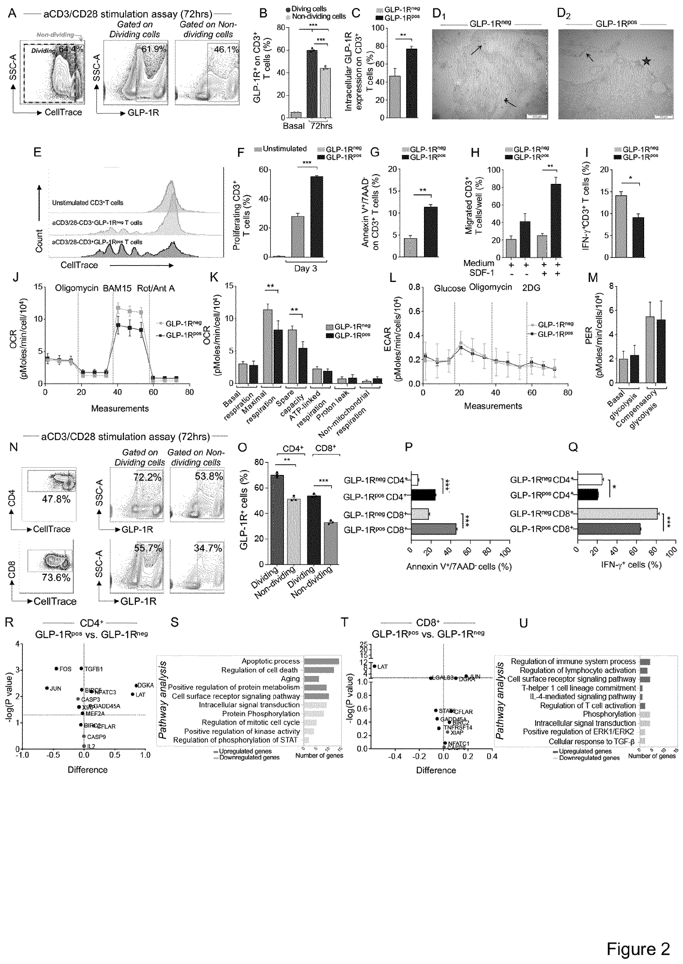

**Figure 2****. GLP-1R^{pos} T cells showed a unique functional profile. A-B:** GLP-1R expression was quantified after 72 hours of stimulation with anti-CD3/CD28 Abs on dividing CD3⁺ T cells as compared to non- dividing CD3⁺ T cells. The quantification of GLP-1R expression in both sub-populations is shown in (B). Experiments were performed in triplicates, and results are representative of one experiment. **C-D₁:** GLP-1R^{neg} CD3⁺ appeared to have a significant intracellular GLP-1R expression as shown by FACS analysis (F) and confirmed by electron microscopy in (D₁) where GLP-1R is present in cellular cytoplasm, predominantly in vesicles (arrows), but is absent in cell membrane, (magnification: X50000 by electron microscopy). **D₂:** GLP1R is present both in cytoplasmic vesicles (arrows) and in cell membrane (stars) of GLP-1R^{pos} CD3⁺ (magnification: X50000). **E-F:** Representative FACS plots of proliferating GLP-1R^{pos} and GLP-1R^{neg} CD3⁺ T cells; the quantification of each related CD3⁺ proliferating T cells is shown in (F). **G:** GLP-1R^{pos} CD3⁺ appeared significantly more apoptotic as compared to their counterparts GLP-1 R^{neg} CD3⁺ T cells. **H:** The percentage of GLP-1R^{pos} CD3⁺T cells migrating to SDF-1 gradient was significantly higher as compared to their counterparts GLP-1R^{neg} CD3⁺ T cells. **I:** Bargraph depicting the quantification of the percentage of IFN-y on GLP-1R^{pos} CD3⁺ and on their counterparts GLP-1R^{neg} CD3⁺ T cells upon their stimulation with soluble anti-CD3/CD28. **J-K:** Higher OCR, maximal respiration and spare capacity after mitochondrial membrane uncoupler BAM15 addition was observed in GLP-1 R^{neg} CD3⁺ T cells as compared to GLP-1 R^{pos} CD3⁺ T cells. **L-M:** Representative profile of the glycolysis stress assay reported as Extracellular acidification rate (ECAR) measurements normalized to cell number, performed on GLP1R^{pos} and GLP1R^{neg} CD3⁺ T cells; the related glycolytic parameters referred as PER measurements normalized to cell number are shown in bargraphs in (M). All respiratory parameters shown in M-N, OP are obtained from 3 independent experiments and data are presented as mean ± standard error of the mean (SEM), a two-way ANOVA test was used to determine statistical significance. **N-O:** GLP-1R expression was depicted after 72 hours of stimulation with anti-CD3/CD28 Abs on dividing CD4⁺ and CD8⁺ T cells as compared to non-dividing CD4⁺ and CD8⁺ T cells; the quantification of GLP-1R expression in all sub-populations is shown in (R). **P:** GLP-1R^{pos} CD4⁺ and CD8⁺ T cells were more apoptotic as compared to their counterparts GLP-1R^{neg} T cells (p<0.001). **Q:** Bargraph showing the quantification of the percentage of IFN-y on GLP-1R^{pos} CD4⁺ and CD8⁺ and on their counterparts GLP-1R^{neg} T cells. **R-U:** Volcano plots and their related pathway analysis showing differently expressed genes in GLP-1R^{pos} CD4⁺ cells versus. GLP-1R^{neg} CD4⁺ T cells and in GLP-1R^{pos} CD8⁺ cells versus. GLP-1R^{neg} CD8⁺ T cells. In S and U, the main biological function/pathway analysis of the data set related to upregulated and downregulated genes on (S) GLP-1R^{pos} CD4⁺ and on (U) GLP-1R^{pos} CD8⁺ T cells are shown. Data are representative of at least n=2 independent experiments. Data are expressed as mean±standard error of the mean (SEM). Data are representative of at least n=3 mice. **P*<0.05; ***P*<0.01; ****P*<0.001. **Abbreviations:** GLP-1R, Glucagon-like peptide 1 receptor; OCR (oxygen consumption rate); SRC (spare respiratory capacity); CT, cell trace; FACS, fluorescence-activated cell sorter; IFN-y, interferon gamma; ECAR, Extracellular acidification rate; PER, Proton Efflux Rate.

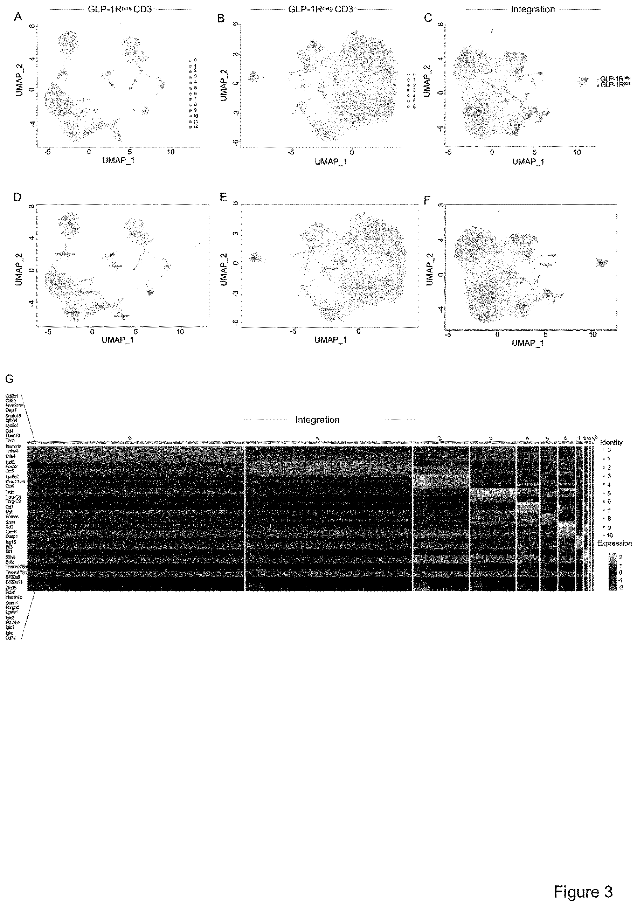

**Figure 3****. scRNAseq unveils a unique molecular profile of GLP-1R^{pos} T cells. A-B:** Uniform manifold approximation and projection (UMAP) for respectively sorted GLP-1R^{pos} and GLP-1R^{neg} CD3⁺ T cells showing 13 cell clusters in the former (A) and 7 main clusters in the latter (B), by using 30 nPCs and 0.5 resolution and 0.2 resolution, respectively. C: UMAP showing the clustering of the integrated dataset. **D-F:** Annotation of different T cell subtypes composing each cluster related to respectively GLP-1R^{pos} and GLP-1R^{neg} CD3⁺ T cells and the integrated dataset is shown. **G:** Heat-map showing the the top marker genes for each cluster within the integrated dataset. Data are representative of respectively n=3 mice and are expressed as mean ± standard error of the mean (SEM). **P*<0.05; ***P*<0.01; ****P*<0.001. **Abbreviations:** scRNAseq, single cell RNA sequencing; GLP-1R, Glucagon-like peptide 1 receptor; UMAP, Uniform manifold approximation and projection.

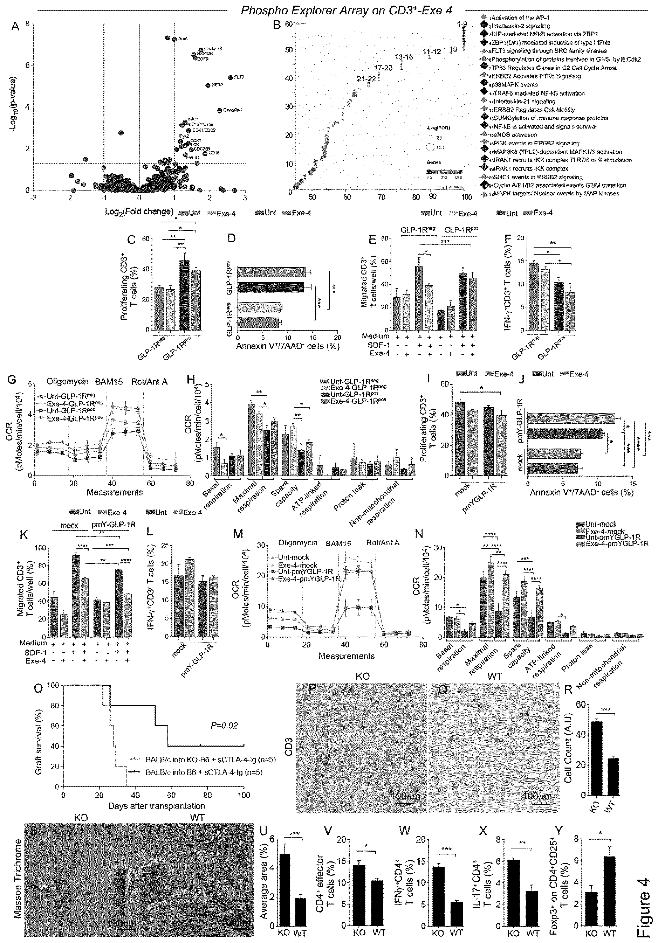

**Figure 4****. GLP-1R signaling modulates T cells activation, anergy and apoptosis. A-B:** Differential profiling analysis of phosphoproteomic array performed on murine CD3⁺ T cells challenged for 24 hours with Exenatide/Exendin 4 (Exe-4) (1 nM) as compared to their counterparts untreated; main differences in phosphoproteomics between the 2 groups of cells are shown as Volcano plot in (A); the buble chart of the reactome anlysis of the main 60 phosphoproteomics dysregulated during Exe-4 treatment is shown in (B). Date represent n=3 samples per conditions. **C:** Exe-4-treatment slightly modulated the proliferation rate of GLP-1R^{pos}, but not of GLP-1R^{neg} CD3⁺ T cells. **D:** GLP-1R^{pos} CD3⁺ T cells appeared significantly more apoptotic as compared to their counterparts GLP-1R^{neg} CD3⁺ T cells, independently of Exe-4 treatment. **E:** Exe-4-treatment reduced the migratory potential of GLP-1R^{neg} CD3⁺ T cells only, with the GLP-1R^{pos} CD3⁺ T cells migrating less. **F:** GLP-1R^{pos} CD3⁺ T cells retained lower IFN-y expression as compared to their negative counterparts. **G-H:** Exe-4 treatment slightly increased the mitochondrial respiration of GLP-1R^{pos} CD3⁺ T cells as revealed by an increase in OCR values at all time points and in maximal OXPHOS. Data are representative of at least n=3/group and are expressed as mean ± standard error of the mean (SEM). Statistical significance was assessed by two-way ANOVA. **P*<0.05; ***P*<0.01; ****P*<0.001; ****P<0.0001. **I:** Exe-4 treatment has no effect on the proliferation of both untransduced-mock and pmY-GLP-1R transduced CD3⁺ T cells. **J:** Exe-4 treatment increased significantly apoptosis in pmY-GLP-1R transduced CD3⁺ T cells only. **K:** Exe-4 treatment reduced the migratory ability in response to SDF-1 in both untransduced-mock and pmY-GLP-1R transduced CD3⁺ T cells. **L:** No effect on IFN-γ expression was observed in both groups in response to Exe-4 treatment. **M-N:** Exe-4 treatment significantly increased OCR values and mitochondrial respiration in pmY-GLP-1R transduced CD3⁺ T cells as compared to untransduced- mock CD3⁺ T cells. Data are representative of at least n=3/group and are expressed as mean ± standard error of the mean (SEM). Statistical significance was assessed by two-way ANOVA. **P*<0.05; ***P*<0.01; ****P*<0.001; *****P*<0.0001. **O:** Heart allograft survival curves from GLP-1R^{-/-} mice or from WT recipients' mice of heart allograft from fully-mismatched BALB/c mice and treated with low-dose CTLA4-lg. **P-R:** Micrographs of CD3 staining in heart allograft sections from either C57BL/6 or GLP-1R KO recipients of BM12 heart allografts; in (R) bar graph shows the relative quantification of heart infiltrating CD3⁺ cells in both groups. **S-U:** Representative Masson-Trichrome histology staining of heart allografts respectively from GLP-1R KO recipients mice or from WT recipients; the average area of fibrosis in heart allografts from both groups of recipients mice is shown in (U). **V:** Bar graph depicting the quantification of CD4⁺ effector-memory splenocytes (defined as CD44⁺ and CD62L-) in GLP-1R KO or WT mice that underwent heart allograft transplantation. **W:** Bar graph depicting the quantification of IFN-γ⁺CD4⁺ T cells in GLP-1R KO or WT mice that underwent heart allograft transplantation. **X:** Bar graph depicting the quantification of IL-17⁺CD4⁺ T cells in GLP-1R KO or WT mice that underwent heart allograft. **Y:** Bar graph depicting the quantification of CD4⁺ regulatory splenocytes (defined as CD25⁺ and FoxP3⁺) in both groups of mice that underwent heart allograft transplantation. Data are representative of at least n=3 mice and are expressed as mean ± standard error of the mean (SEM). **P*<0.05; ***P*<0.01; ****P*<0.001; *****P*<0.0001. **Abbreviations:** GLP-1R, Glucagon-like peptide 1 receptor; Exe-4, exendin-4; Exe-9, exendin-9-39; FACS, fluorescence-activated cell sorter scan; BAZ2A, *Bromodomain Adjacent To Zinc Finger Domain 2A;* CCG8, Voltage-dependent calcium channel gamma-8 subunit; WT, wild type; IFN-y, interferon gamma; CTLA4, cytotoxic T-lymphocyte antigen 4; BAM15, (N5,N6-bis(2-Fluorophenyl)[1,2,5]oxadiazolo[3,4-b]pyrazine-5,6-diamine); FoxP3, forkhead box P3.

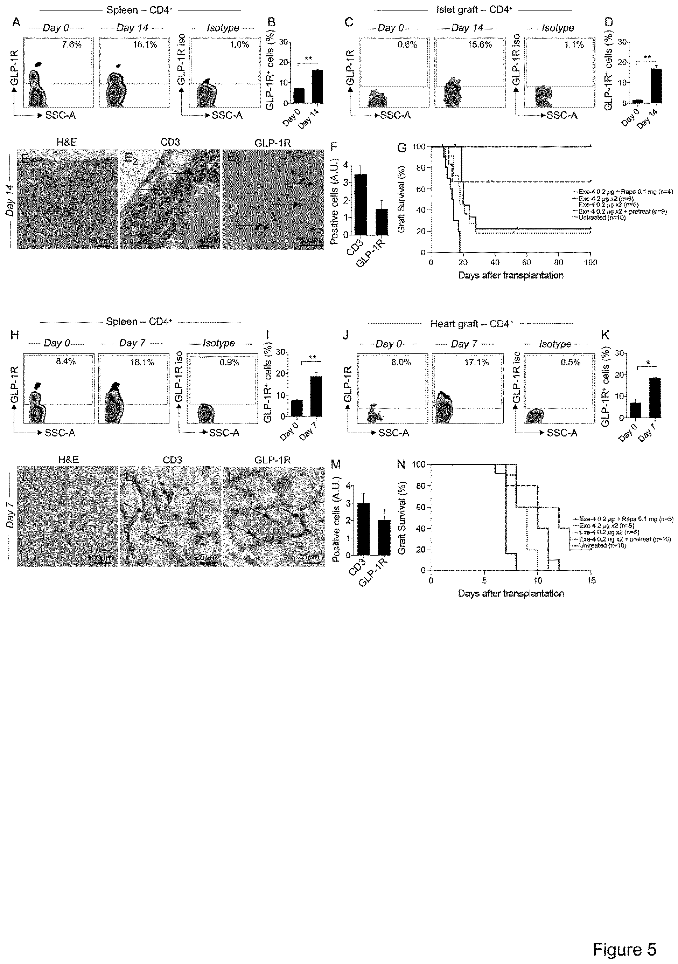

**Figure 5****. GLP-1R signaling prolongs allograft survival. A:** Representative zebra plot of GLP-1R expression by splenic murine CD4⁺ T lymphocytes in basal condition and after islet allotransplantation; plots are associated to related isotype control. **B:** Bar graph representing quantification of GLP-1R expression by spleen CD4⁺ lymphocytes in basal condition and after islet allotransplantation. **C:** Representative zebra plot of GLP-1R expression by murine kidney CD4⁺ T lymphocytes in basal condition and after islet allotransplantation under kidney capsule; the plots are associated to related isotype control. **D:** Bar graph representing quantification of GLP-1R expression by CD4⁺ lymphocytes in normal kidneys and after islet allograft. **E₁:** Hematoxylin and eosin (H&E) histology staining depicts the immune infiltrate within the islet allograft at day 14. **E₂:** Islet allograft immunohistochemistry for CD3⁺ cells (arrows). **E₃:** Islet allograft immunohistochemistry for GLP-1R^{pos} cells (arrows). **F:** Bar graph displaying the quantification of CD3⁺ and GLP-1R^{pos} cells in islet allograft. **G:** Islet allograft survival curves from mice treated with different doses of the GLP-1R agonist Exenatide (dashed line) or saline (continuous line). Mice underwent islet allograft and were then treated with saline, Exenatide 2µg twice/day, or both Exenatide and Exendin 9-39 2µg twice/day. **H:** Representative zebra plot of GLP-1R expression by splenic murine CD4⁺T lymphocytes obtained from mice in basal condition or after heart allotransplantation; the plots are associated to related isotype control. **I:** Bar graph representing quantification of H. **J:** Representative zebra plot of GLP-1R expression by heart allograft infiltrating CD4⁺ T lymphocytes obtained from mice in basal condition or after heart allotransplantation; the plots are associated to related isotype control. **K:** Bar graph representing the quantification of (J). **L₁:** Hematoxylin and eosin (H&E) histology staining depicts the immune infiltrate within the heart allograft at day 7. **L₂:** Heart allograft immunohistochemistry for CD3⁺ cells (arrows). **L₃:** Heart allograft immunohistochemistry for GLP-1R^{pos} cells (arrows). **M:** Bargraph displaying the quantification of CD3⁺ and GLP-1R^{pos} cells in heart allograft. **N:** Heart allograft survival curves from mice treated with different doses of the GLP-1R agonist Exenatide (dashed line) or saline (continuous line). C57BL/6 mice underwent heart allograft (day 7); mice were treated with saline, Exenatide 2µg twice/day, or both Exenatide and Exendin 9-39 2µg twice/day. Data are representative of at least n=3 mice and are expressed as mean ± standard error of the mean (SEM). **P*<0.05; ***P*<0.01; ****P*<0.001. **Abbreviations:** GLP-1R, Glucagon-like peptide 1 receptor; FACS, fluorescence-activated cell sorter scan; H&E, Hematoxylin and eosin.

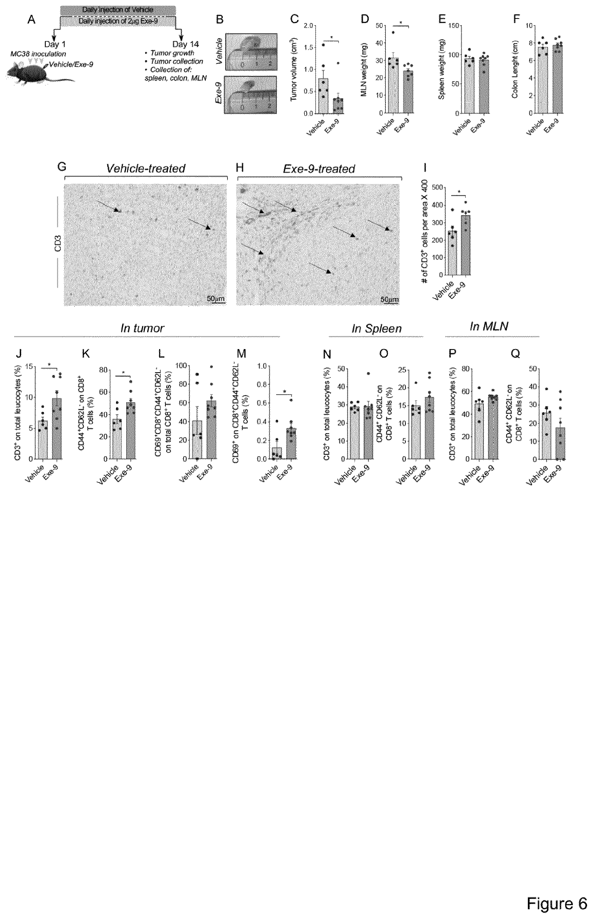

**Figure 6****. GLP-1R antagonism triggers an antitumor immunity. A:** Schematic overview of the study protocol. **B:** Representative pictures of tumors extracted from respectively MC38 tumor-bearing mice treated with vehicle (PBS) or treated with Exe-9 at day 14. **C-F:** Quantitative bargraph depicting tumor volume, mesenteric lymph nodes (MLN) weight, spleen weight and colon weight of vehicle-treated or Exe-9-treated mice. **G-I:** Representative immunohistochemical images showing intratumoral CD3 staining from vehicle-treated or Exe-9-treated mice, and their related quantification is shown in **(I)**. **J-M:** Quantitative bargraph depicting FACS quantification of tumor infiltrating CD3⁺ T cells, CD8⁺ effector memory T cells, activated CD8⁺ T cells within total CD8⁺ T cells (L) or within CD8⁺ effector memory T cells (M), extracted from vehicle-treated or Exe-9-treated mice. **N-Q:** Quantitative bargraph depicting FACS quantification of total splenic CD3⁺ T cells and CD8⁺ effector memory T cells or CD3⁺ T cells and total CD8⁺ effector memory T cells extracted from MLNs of vehicle-treated or Exe-9-treated mice. Data are representative of respectively n=6 and n=8 samples from Vehicle-treated and Exe-9-treated mice and are expressed as mean ± standard error of the mean (SEM). **P*<0.05; ***P*<0.01; ****P*<0.001. **Abbreviations:** GLP-1R, Glucagon-like peptide 1 receptor; Abs, antibodies; FACS, fluorescence-activated cell sorter scan; MLNs, mesenteric lymph nodes.

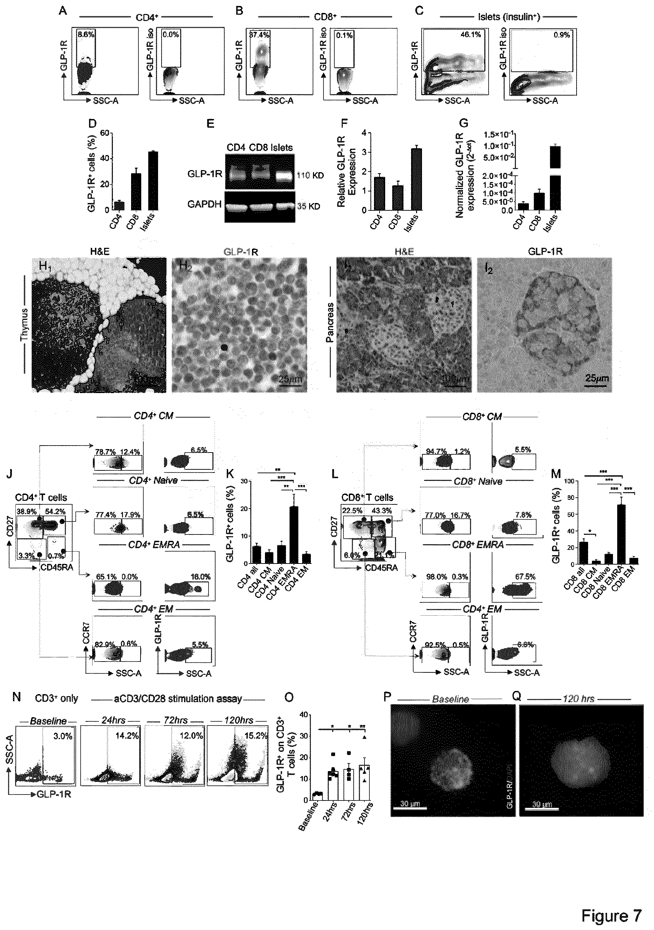

**Figure 7****. GLP-1R is relevant for human T cells. A-B:** Representative dot plot of GLP-1R expression by human circulating CD4⁺ and CD8⁺ cells and associated isotype controls. **C:** representative dot plots of GLP-1R expression by human pancreatic β-cells (positive control) and associated isotype controls. **D:** Bar graph depicts results of flow cytometric analysis of GLP-1R expression by human CD4⁺ and CD8⁺ T lymphocytes, together with pancreatic islets as positive control. **E-F:** Western blot analysis and associated quantification confirm GLP-1R expression on CD4⁺ and CD8⁺ T lymphocytes; pancreatic islets represent the positive control. **G:** Bar graph representing normalized mRNA expression of GLP-1R in human CD4⁺ and CD8⁺ T lymphocytes; pancreatic islets represent the positive control. **H₁-H₂:** Representative images of hematoxylin and eosin histology staining and immunoreactive GLP-1R^{pos} cells in human thymus. **I₁-I₂**. Hematoxylin and eosin histology staining, and immunoreactive GLP-1R^{pos} cells in human pancreas. **J:** Representative dot plots for gating strategy and GLP-1R expression by CD4⁺CD45RA⁺CD27⁺CCR7⁺ naive T cells, CD4⁺CD45RA⁻CD27⁺CCR7⁺ central memory T cells, CD4⁺CD45RA⁻CD27⁻CCR7⁻ effector memory T cells and CD4⁺CD45RA⁺CD27⁻CCR7⁻ terminally differentiated effector memory T cells. **K:** Quantification of flow cytometric analysis from (J) and comparison with whole CD4⁺ population. **L:** Representative dot plots for gating strategy and GLP-1R expression by CD8⁺CD45RA⁺CD27⁺CCR7⁺ naive T cells, CD8⁺CD45RA⁺CD27⁺CCR7⁺ central memory T cells, CD8⁺CD45RA⁺CCR7⁻ effector memory T cells and CD8⁺CD45RA⁺CD27⁻CCR7⁻ terminally differentiated effector memory T cells. **M:** Quantification of flow cytometric analysis from L and comparison with whole CD8⁺ population. **N-O:** Cell trace violet-labelled CD3⁺ T cells were activated with anti-CD3/CD28 Abs. After 24 hours, 72 hours and 120 hours, the cells were harvested and stained for GLP-1R; representative dot plots for FACS anlysis related to each time point (24, 72 and 120 hours) are shown in (N) and their corresponding bargraphs are shown in (O). **P:** Immunofluorescence staining depicting GLP-1R expression on human CD3⁺ T cells at baseline. **Q:** Immunofluorescence staining depicting GLP-1R expression on human CD3⁺ T cells stimulated for 120 hours with soluble anti-CD3/CD28 Abs. Data are representative of at least n=3 samples and are expressed as mean ± standard error of the mean (SEM). **P*<0.05; ***P*<0.01; ****P*<0.001. **Abbreviations:** GLP-1R, Glucagon-like peptide 1 receptor; Abs, antibodies; FACS, fluorescence-activated cell sorter scan; EM, effector memory; CM, central memory; EMRA, terminally differentiated effector memory expressing CD45RA.

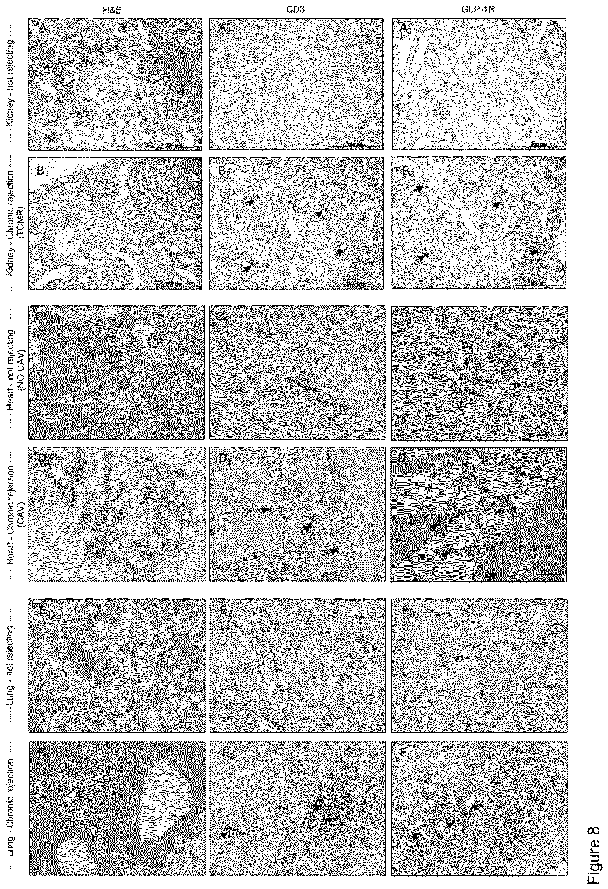

**Figure 8****. Expansion of GLP-1R^{pos} T cells during human allograft rejection. A₁-A₃:** Hematoxylin and eosin, CD3 and GLP-1R stainings on human renal biopsies from kidney-transplanted non-rejecting cases, (magnification: 20X, scale bar: 200 µm). **B₁**-**Bs:** Hematoxylin and eosin, CD3 and GLP-1R stainings on human renal biopsies from kidney-transplanted TCMR-rejecting cases, (magnification: 10X, scale bar: 200 µm). **C₁**-**C₃:** Hematoxylin and eosin, CD3 and GLP-1R stainings on human heart biopsies from heart-transplanted non-CAV cases, (magnification: 40X, scale bar: 1 nm). **D₁-D₃:** Hematoxylin and eosin, CD3 and GLP-1R stainings on human heart biopsies from heart-transplanted CAV cases, (magnification: 40X, scale bar: 1nm). **E₁-E₃:** Hematoxylin and eosin, CD3 and GLP-1R stainings on human lungs biopsies from non-transplanted cases, (magnification: 40X, scale bar: 1nm). **F₁-F₃:** Hematoxylin and eosin, CD3 and GLP-1R stainings on human lungs biopsies from lung-transplanted chronic rejecting cases, (magnification: 40X, scale bar: 1nm). Data are representative of at least n=2 samples and are expressed as mean ± standard error of the mean (SEM). *P<0.05; **P<0.01; ***P<0.001. **Abbreviations:** GLP-1R, Glucagon-like peptide 1 receptor; TCMR, T cell-mediated rejection; CAV, chronic allograft vasculopathy.

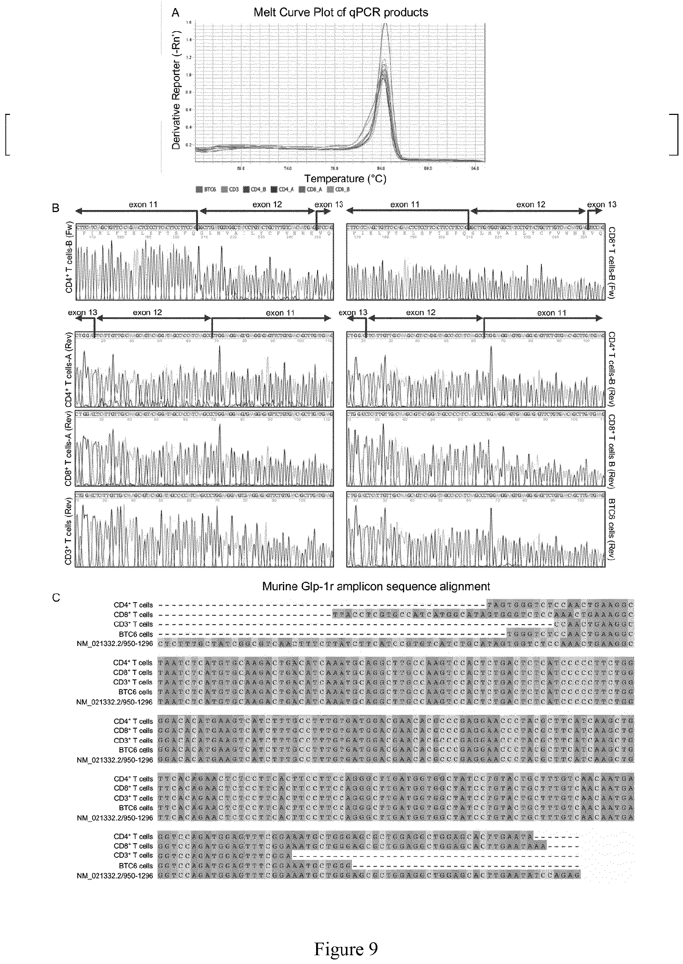

**Figure 9****. A:** Melting curve plots of qRT-PCR products showing a single peak at ~84°C confirming a unique amplification product obtained when the cDNA of BTC6, respectively 2 biological replicates of murine CD4⁺ and CD8⁺ T cells and CD3⁺ T cells was used. **B:** Representative electropherograms showing the 5'-to-3' sequence of the amplified *Glp-1r* region spanning exons 11 to 13 in murine CD4⁺, CD8⁺, CD3⁺ T cells and BTC6 cells obtained by Sanger sequencing. **C:** Sequence alignment of nucleotides 950-1296 of murine *Glp-1r* mRNA reference sequence NM_021332.2 to that of the qRT-PCR products obtained from RNA of CD4⁺, CD8⁺, CD3⁺ T cells and of BTC6 cells, further confirming the amplification of *Glp-1r* in these cells. **Abbreviations:** BTC6, Beta-TC-6 *cell* line; qRT-PCR, Two-step quantitative reverse transcriptase-PCR; cDNA, complementary DNA.

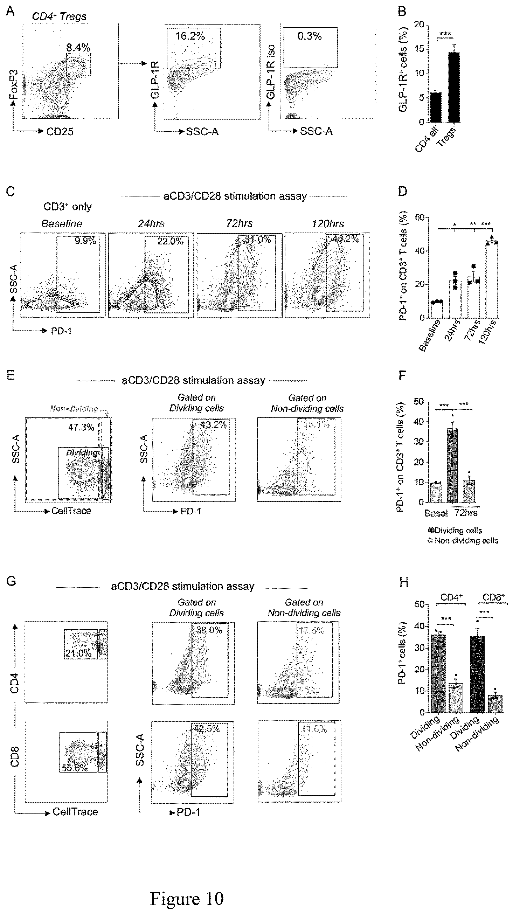

**Figure 10****. A** Representative dot plots for gating strategy and GLP-1R expression by CD4⁺CD25⁺FoxP3⁺ regulatory cells. **B:** Quantification of flow cytometric analysis from A and comparison with whole CD4⁺ population. **C-D:** Cell trace violet-labelled CD3⁺ T cells were activated with anti-CD3/CD28 Abs. After 24 hours, 72 hours and 120 hours cells were harvested and stained for PD-1; representative dot plots for FACS analysis related to each time point (24, 72 and 120 hours) are shown in (C) and their corresponding bargraphs are shown in (D). **E-F:** PD-1 expression was depicted after 72 hours of stimulation with anti-CD3/CD28 Abs on dividing CD3⁺ T cells as compared to non-dividing CD3⁺ T cells; the quantification of PD-1 expression in both sub-populations is shown in (F). **G-H:** PD-1 expression was depicted after 72 hours of stimulation with anti-CD3/CD28 Abs on dividing CD4⁺ and CD8⁺ T cells as compared to non-dividing CD4⁺/CD8⁺ T cells, the quantification of PD-1 expression in both sub-populations is shown in (H). **Abbreviations:** GLP-1R, Glucagon-like peptide 1 receptor; FoxP3, forkhead box P3; Abs, antibodies; FACS, fluorescence-activated cell sorter scan; CT, cell trace.

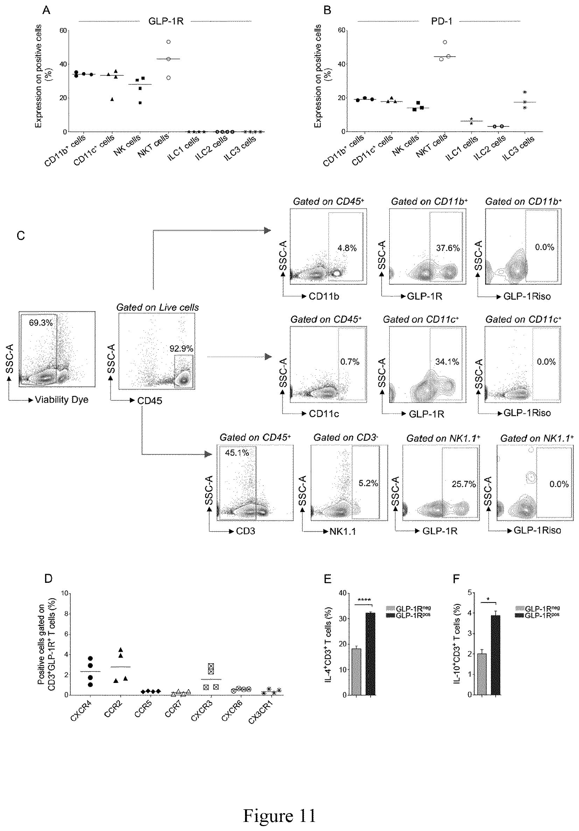

**Figure 11****. A-C:** A head to-head comparison of GLP-1R and PD-1 expression on cells of innate immunity. **A:** GLP-1R expression was assessed by FACS analysis on respectively CD11b⁺ cells, CD11c⁺ cells, NK cells defined as CD3-/NK1.1⁺, NKT cells defined as CD4⁺/NK1.1⁺/CD44⁺/CD127⁺ and by ILCs (ILC1: NK1.1⁺/CD127⁺/CD49a⁺/CD335⁺; ILC2: CD117⁺/CD127⁺/IL33R⁺, ILC3: CD117⁺/CD127⁺/IL-23R⁺). **B:** PD-1 expression by the aforementioned cells of innate immunity are shown. **C:** Gating strategy delineating the expression of GLP-1R on CD11b⁺, CD11c⁺ and NK cells of innate immunity. **D:** The profile of several chemokine receptors (CXCR4, CCR2, CCR5, CCR7, CXCR3, CXCR6 and CX3CR1) was explored on GLP-1R^{pos} CD3⁺ T cells. **E-F:** IL-4 and IL-10 expression by GLP-1R^{pos} CD3⁺ T cells. **Abbreviations:** GLP-1R, Glucagon-like peptide 1 receptor; Abs, antibodies; FACS, fluorescence-activated cell sorter scan; ILCs,-Innate Lymphoid Cells.

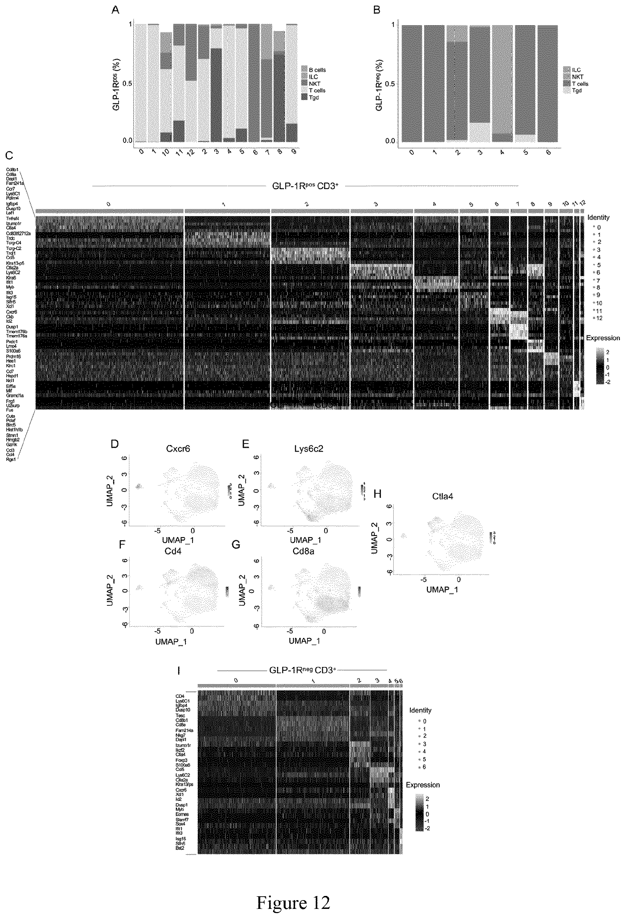

**Figure 12****. A:** Barplot of the sorted GLP-1R^{pos} CD3⁺ T cells showing the SingleR annotation for the 13 identified clusters, using 30 nPCs and 0.5 resolution. **B:** Barplot of the sorted GLP-1R^{neg} CD3⁺ T cells showing the SingleR annotation for the 7 identified clusters, using 30 nPCs and 0.2 resolution. **C:** Heat-map showing the most relevant genes present within the GLP-1R^{pos} CD3⁺ subset. **D-H:** UMAPs delineating the main gene/markers identified in the GLP-1R^{neg} CD3⁺ T cell-scRNAseq dataset. **I:** Heat-map showing the most relevant genes present within the GLP-1R^{neg} CD3⁺ subset. **Abbreviations:** GLP-1R, Glucagon-like peptide 1 receptor; ATP, adenosine triphosphate; UMAP, Uniform manifold approximation and projection; scRNAseq, single cell RNA sequencing.

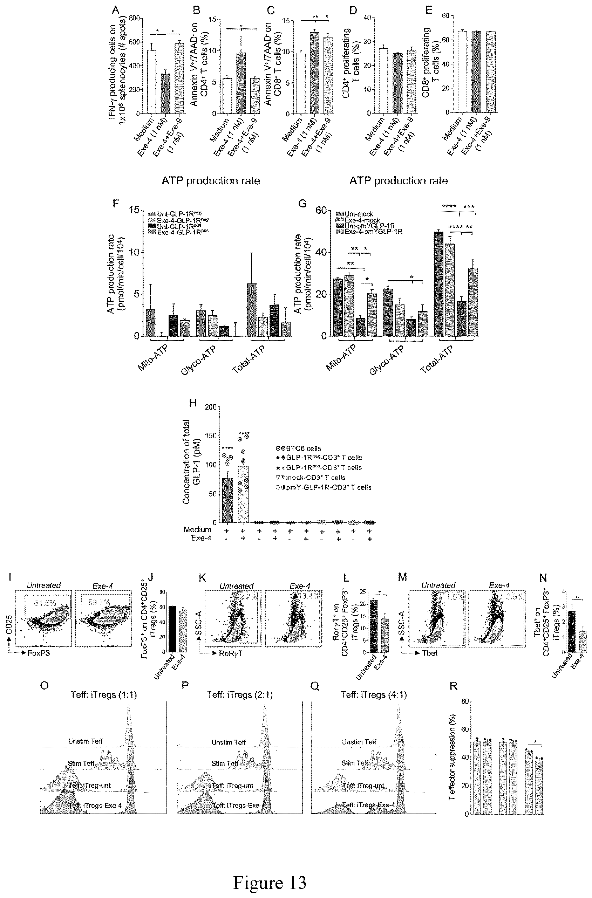

**Figure 13****. A:** Exe-4 significantly reduced the number of IFN-y producing cells by mitogen-stimulated splenocytes, which was dampened upon co-culture with GLP-1R antagonist Exendin-9-39 (Exe-9) (one way-ANOVA, *P*<0.05). **B-C:** Exe-4 treatment induced cell death on activated CD4⁺ and CD8⁺ T cells (*p*<0.05 in Exe-4 (1 nM) treated CD4⁺ T cells vs. medium, *p*<0.01 in medium vs. Exe-4 (1 nM) treated CD4⁺ T cells). **D-E:** No differences were observed comparing the percentages of proliferating (or cell trace positive) CD4⁺ and CD8⁺ T cells stimulated with anti-CD3/CD28 Abs upon their challenge with Exe-4 neither with the addition of the antagonist Exe-9. **F-G:** ATP-production rates on the 2 subpopulations of untreated GLP-1R^{neg} and GLP-1R^{pos} CD3⁺ T cells as compared to those treated with Exe-4 (1 nM) and on untransduced (mock) CD3⁺ T cells and pmY-GLP-1R-transduced CD3⁺ T cells as compared to those treated with Exe-4 (1 nM). **H:** Total GLP-1 secretion/production by GLP-1R^{pos} and GLP-1R^{neg} CD3⁺ T cells and by untransduced mock and pmY-GLP-1R CD3⁺ T cells in response to Exe-4 as compared to that of BTC6 cell line, used here as a positive control. **I-J:** Representative FACS dot plots showing the percentage of FoxP3⁺CD4⁺CD25⁺ Tregs during Treg induction assay in response to Exe-4 treatment; the quantitative bargraph is shown in (J). **K-L:** Representative FACS dot plots showing the percentage of Ror-yT on FoxP3⁺CD4⁺CD25⁺ Tregs during Treg induction assay in response to Exe-4 treatment; the quantitative bargraph is shown in (L). **M-N:** Representative FACS dot plots showing the percentage of Tbet on FoxP3⁺CD4⁺CD25⁺ Tregs during Treg induction assay in response to Exe-4 treatment; the quantitative bargraph is shown in (N). **O-R:** Representative FACS histograms reflecting effector T cell suppression shown as CTV dilution upon co-culture of untreated-iTregs or Exe-4-treated iTregs at respectively the following ratios: 1:1, 2:1 and 4:1; quantitative bargraph reflecting the percentage of effector T cell suppression is shown in (R). **Abbreviations:** GLP-1R, Glucagon-like peptide 1 receptor; FACS, fluorescence-activated cell sorter scan; IFN-y, interferon gamma; FoxP3, forkhead box P3; CTV; cell trace violet; iTregs; induced Tregulatory cells.

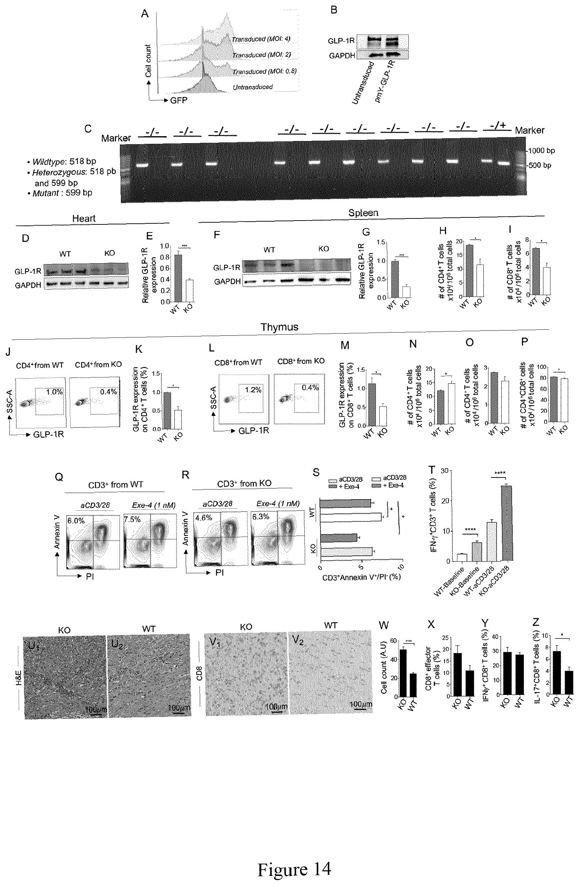

**Figure 14****. A-B:** GLP-1R upregulation is verified by respectively FACS analysis (A) and western blot analysis (B), in pmY-GLP-1R-transduced CD3⁺ T cells as compared to their counterparts untransduced. **C:** The baseline phenotype of GLP-1R KO mice is shown on agarose gel, where Wildtype are identified by the presence of 518 bp band, Heterozygous by the presence of respectively 518 pb and 599 bp bands and the Mutant allele is distinguished by the presence of 599 bp band. **D-E:** Knockdown of GLP-1R within heart was confirmed by western blot analysis. **F-I:** Knockdown of GLP-1R within spleen of GLP-1R KO mice was confirmed by western blot analysis; the absolute numbers of CD4⁺ and CD8⁺ T cells were assessed by FACS analysis within spleen of GLP-1R KO mice as compared to WT mice and shown in (H and I). **J-P:** The knockdown of GLP-1R was confirmed in CD4/CD8 T cells in the thymus of GLP-1R KO mice; the absolute numbers of respectively CD4⁺, CD8⁺ and CD4⁺CD8⁺ T cells were assessed by FACS analysis within the thymus of GLP-1R KO mice as compared to WT mice and shown in (N-P). **Q-S:** Apoptosis assay of CD3⁺ T cells from GLP-1R KO mice at baseline and after mitogenic stimulation as compared to their counterparts from WT mice, the quantification of apoptotic cells in both groups and at baseline and/or after mitogen stimulation is shown in (S). **T:** Quantitative bargraph depicting the percentage of splenic IFN-γ⁺CD3⁺ T cells from GLP-1R KO mice as compared to WT mice at baseline and in response to mitogen stimulation. **U₁-U₂:** Hematoxylin and eosin staining in heart allografts obtained from C57BL/6 WT mice transplanted with BALB/c hearts and treated with low dose CTLA4-lg and from C57BL/6 GLP-1R KO mice recipients of BALB/c hearts and treated with low dose CTLA4-lg, (magnification 20X, and scale bar 100 µm). **V₁-V₂** and **W:** CD8 staining in heart allografts obtained from C57BL/6 WT mice transplanted with BALB/c hearts and treated with low dose CTLA4-lg and from C57BL/6 GLP-1R KO mice recipients of BALB/c hearts and treated with low dose CTLA4-lg, (magnification 20X, and scale bar 100 µm); the relative quantification of CD8 infiltrating cells in both groups of transplanted mice is shown in (W). **X-Z:** Bargraphs depicting the quantification of respectively CD8 effector T cells as well as the significant increase in the percentages of IL-17⁺ CD8⁺ T cells in splenocytes from both groups of transplanted mice as aforementioned. **Abbreviations:** GLP-1R, Glucagon-like peptide 1 receptor; Exe-4, Exendin 4/Exenatide; WT; wildtype.

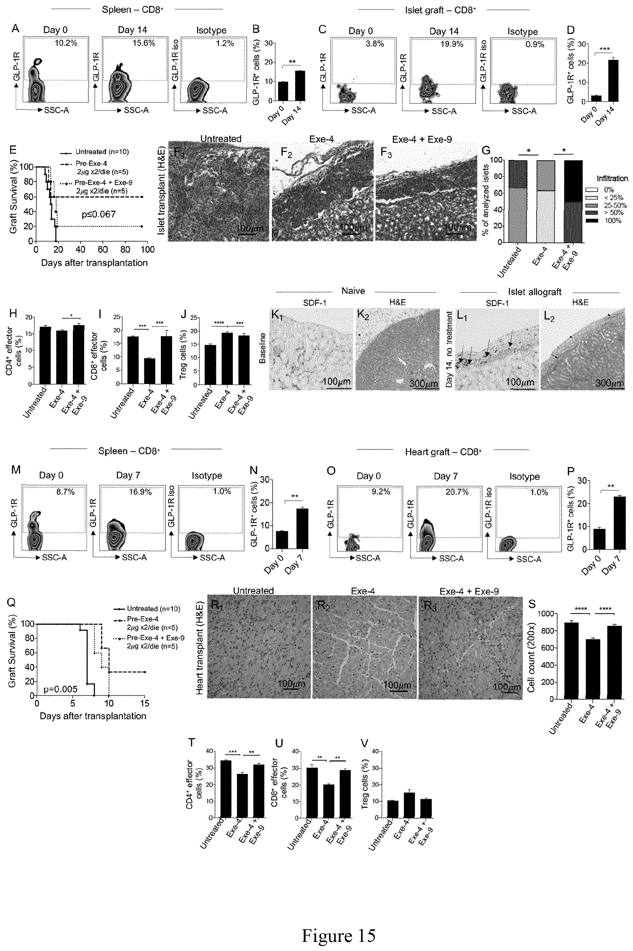

**Figure 15****. A:** Representative zebra plot of GLP-1R expression by splenic murine CD8⁺ T lymphocytes in basal condition and after islet allotransplantation; FACS plots are associated to related isotype control. **B:** Bargraph representing quantification of GLP-1R expression by spleen CD8⁺ lymphocytes in basal condition and after islet allotransplantation. C: Representative zebra plot of GLP-1R expression by murine CD8⁺ T lymphocytes in basal condition and after islet allotransplantation under kidney capsule; the plots are associated to related isotype control. **D:** Bargraph representing quantification of GLP-1R expression by CD8⁺ lymphocytes in normal kidneys and after islet allograft. **E:** Islet allograft survival curves from mice treated with GLP-1R agonist Exenatide (Exe-4, dashed line), a combination of Exe-4 and Exendin 9-39 (dotted line) or saline (dashed line); statistical analysis was performed using Student's t-test with Welch's correction (**p*<0.05). **F₁-F₃:** Representative hematoxylin and eosin histology staining of day-14 islet allografts from mice treated with saline, Exenatide 2µg twice/day, or both Exenatide and Exendin 9-39 2µg twice/day. **G:** Immune infiltrate quantification after hematoxylin and eosin histology staining in islet allografts from mice treated with saline, Exenatide 2µg twice/day, or both Exenatide and Exendin 9-39 2µg twice/day. **H-J:** Bargraph depicting the quantification of splenic CD4⁺ effector-memory, CD8⁺ effector-memory T cells or splenic CD4⁺ regulatory T cells in mice who underwent islet allograft and were treated with saline, Exenatide 2µg twice/day, or both Exenatide and Exendin 9-39 2µg twice/day.**K₁** Representative immunohistochemistry staining for SDF-1 kidney at baseline, (magnification: 10X, scale bar: 300 µm). **K₂**: Representative H&E histology staining on kidney at baseline (magnification: 10X, scale bar: 300 µm). **L_{1:}** Representative immunohistochemistry staining for SDF-1 on islet allograft at day 14 post-transplant; the positive immunosignal (arrows) in the islet implant area is located in the inflammatory foci, (magnification: 20X, scale bar: 300 µm). **L₂:** Representative H&E histology staining on islet allograft at baseline and at day 14 post-transplant, the islet implant site is thickened by a collagen fiber deposition encompassing both inflammatory foci and islet epithelial cells (magnification: 10X, scale bar: 300 µm). **M:** Representative FACS zebra plot of GLP-1R expression by splenic murine CD8⁺ T lymphocytes in basal condition and after heart allotransplantation; the plots are associated to related isotype control. **N:** Bargraph representing quantification of GLP-1R expression by spleen CD8⁺ lymphocytes in basal condition and after heart allotransplantation. **O:** Representative zebra plot of GLP-1R expression by murine heart infiltrating CD8⁺T lymphocytes in basal condition and in heart allograft; the plots are associated to related isotype control. **P:** Bargraph representing quantification of GLP-1R expression by heart infiltrating CD8⁺ lymphocytes in basal condition and in heart allograft. **Q:** Heart allograft survival curves from mice treated with GLP-1R agonist Exenatide/Exendin-4 (Exe-4, dashed line), a combination of Exe-4 and Exendin 9-39 (dotted line) or saline (dashed line); statistical analysis was performed using Student's t-test with Welch's correction (*p<0.05). **R₁-R₃:** Representative hematoxylin and eosin (H&E) histology staining of day-7 heart allograft from mice treated with saline, Exenatide 2µg twice/day, or both Exenatide and Exendin 9-39 2µg twice/day. **S:** Immune infiltrate quantification after hematoxylin and eosin histology staining in heart allografts from mice treated with saline, Exenatide 2µg twice/day, or both Exenatide and Exendin 9-39 2µg twice/day. **T-V:** Bar graph depicting the quantification of splenic CD4⁺ effector-memory and CD8⁺ effector-memory T cells or splenic CD4⁺ regulatory T cells in mice who underwent heart allograft and were treated with saline, Exenatide 2µg twice/day, or both Exenatide and Exendin 9-39 2µg twice/day. **Abbreviations:** GLP-1R, Glucagon-like peptide 1 receptor; FACS, fluorescence-activated cell sorter scan; Exe-4, Exenatide or exendin-4; FoxP3, forkhead box P3.

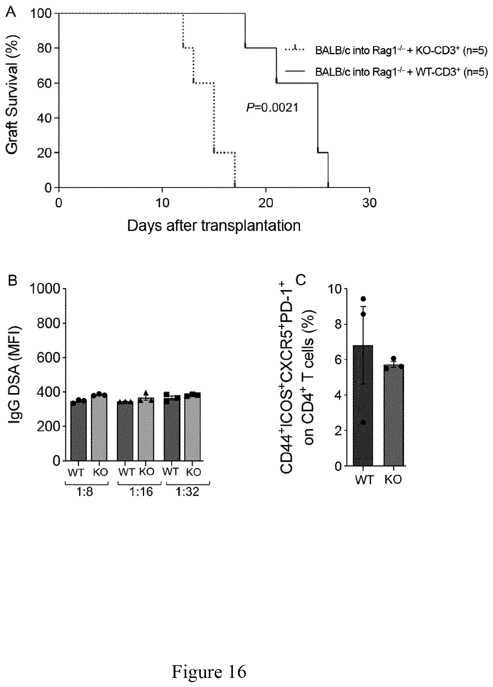

**Figure 16****. A:** Heart allograft survival curves from Rag1^{-/-} mice that received heart allograft from BALB/c mice and were adoptively transferred with either CD3⁺ T cells from GLP-1R KO mice or from WT mice; statistical analysis was performed using Student's t-test with Welch's correction (*p<0.05). **B:** Donor specific alloantibody levels (DSA) shown here as MFI in WT mice or from GLP-1R KO mice that received a heart allograft from BALB/c mice. **C:** Quantitative bargraph showing the percentage of circulating T follicular helper cells were assessed on PBMCs from WT mice or from GLP-1R KO mice that received a heart allograft from BALB/c mice. **Abbreviations:** GLP-1R, Glucagon-like peptide 1 receptor; WT, wild type; DSA; donor specific alloantibody; MFI, mean fluorescence intensity; cTfh; circulating follicular helper T cells; PBMCs, peripheral blood mononuclear cells.

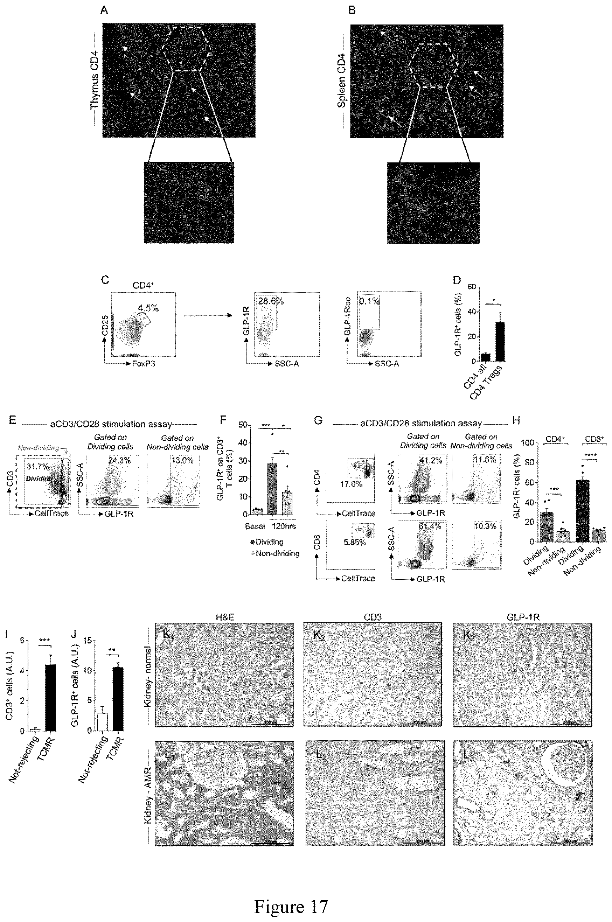

**Figure 17****. A-B:** Images depicting Chromogenic in situ hybridization of GLP-1R on thymus, GLP-1R mRNA in human thymus and spleen and co-localization with CD4. **C:** Representative dot plots for gating strategy and GLP-1R expression by CD4⁺CD25⁺FoxP3⁺ regulatory cells. **D:** Quantification of flow cytometric analysis from (C) and comparison with whole CD4⁺ population. **E-F:** GLP-1R expression was depicted after 72 hours of stimulation with anti-CD3/CD28 Abs on dividing human CD3⁺ T cells or Cell trace Violet⁺ cells as compared to non-dividing CD3⁺ T cells; the quantification of GLP-1R expression in both sub-populations is shown in (F). **G-H:** GLP-1R expression was depicted after 72 hours of stimulation with anti-CD3/CD28 Abs on dividing non-dividing CD4⁺/CD8⁺ T cells, the quantification of GLP-1R expression in both sub-populations is shown in (H); experiments were performed in triplicates, and results are representative of one experiment. **I-J:** The quantification of CD3⁺/GLP-1R⁺ in T cell-mediated-rejecting cases (TCMR) and no-rejecting cases is shown. **K₁-K₃:** Hematoxylin and eosin, CD3 and GLP-1R staining on human renal biopsies from normal kidney cases, (magnification: 20X, scale bar: 200 µm). **L₁-L₃:** Hematoxylin and eosin, CD3 and GLP-1R staining on human renal biopsies from antibody-mediated-rejecting cases (AMR), (magnification: 10X, scale bar: 200 µm). **Abbreviations:** GLP-1R, Glucagon-like peptide 1 receptor; FACS, fluorescence-activated cell sorter scan; AMR, antibody-mediated rejection.

"Tumor" and "cancer" are herein used as synonyms.

For "prevention" is intended that administration of the agent decreases the chance of developing a disease or condition, i.e. it decreases the chance of developing a cancer. In some embodiments, for "prevention" is intended that administration of the agent stops or slows down progression of a disease that has already begun. For example, in some embodiments the agent of the invention is administered to a subject who already has cancer and the cancer does not develop to a more advanced stage.

For "treatment" is intended that administration of the agent improves or cures or reverts a condition or a disease, i.e. it improves or cures or reverts a cancer. In some embodiments, for "treatment" it is intended that the disease, such as cancer, is not completely cured but it reverts to a less advanced stage.

According to the present invention, the tumor can be any tumor, in particular it can be a solid or a liquid tumor.

Preferably, said tumor is a solid tumor selected from the group consisting of gastrointestinal cancer, colorectal cancer, pancreatic cancer, lung cancer, breast cancer, bladder cancer, prostate cancer, testicular cancer, ovarian cancer, uterine cancer, stomach cancer, liver cancer, renal cancer, melanoma, retinoblastoma, neuroblastoma, and sarcoma, or a liquid tumor selected from the group consisting of leukemias, lymphoma, and multiple myeloma.

For GLP-1R is intended Glucagon Like Peptide-1 receptor. For example, as described in (21-23).

For GLP-1R antagonist is intended a molecule able to inhibit and/or block at least partially the activity of GLP-1R, in particular able to inhibit and/or block at least partially the activation of the downstream pathway mediated by GLP-1R. For example, said molecule may reduce the activation of GLP-1R, modulate the binding of GLP-1R endogenous agonists or ligands or otherwise block GLP-1R. A GLP-1R antagonist may decrease activation of GLP-1R by at least 20%, at least 50%, at least 90% or 100%. Any GLP-1R antagonist known in the field can be used according to the present invention.

An exemplary GLP-1R antagonist is a molecule able to bind to GLP-1R⁺ cells and CD3⁺ T cells, resulting in T-cell activation and cytotoxic T-cell response, preferably a bispecific single-chain molecule. Such molecule may be synthetized by the skilled person according to common general knowledge in the field.

Preferred GLP-1R antagonists are Exendin fragment (9-39), Exendin-3 (9-39) amide, GLP-1R Antagonist 1 (compound 5d), TB-001-003, TB-222-023 or Avexitide, VU 0650991, and GLP-1 (9-36) amide. All such compounds are known in the art and commercially available. See for example the following references:
Exendin-3 (9-39) amide (24): Montrose-Rafizadeh C, Yang H, Rodgers BD, Beday A, Pritchette LA, Eng J. High potency antagonists of the pancreatic glucagon-like peptide-1 receptor. J Biol Chem. 1997 Aug 22;272(34):21201-6. doi: 10.1074/jbc.272.34.21201. GLP-1R Antagonist 1 (compound 5d) (25, 26): Kellie D. Nance, et al. Discovery of a novel series of orally bioavailable and CNS penetrant Glucagon-Like Peptide 1 Receptor (GLP-1R) non-competitive antagonists based on a 1,3-disubstituted-7-aryl-5,5-bis(trifluoromethyl)-5,8-dihydropyrimido[4,5-d]pyrimidine-2,4(1H,3H)-dione core. J. Med. Chem. 19 Jan 2017.
TB-001-003 (26): Optimization of a Glucagon-Like Peptide 1 Receptor Antagonist Antibody for Treatment of Hyperinsulinism. Sean M. Peterson, Christine A. Juliana, Cameron F. Hu, Jinghua Chai, Carson Holliday,1 Kara Y. Chan,1 Ana G. Lujan Hernandez, Zoe Challocombe, Linya Wang, Zhen Han, Nikhil Haas, Ryan Stafford, Fumiko Axelrod, Tom Z. Yuan, Diva D. De Leon and Aaron K. Sato. Diabetes 2023;72:1320-1329. https://doi.org/10.2337/db22-1039.
TB-222-023 or Avexitide (27, 28): De León DD, et al. Exendin-(9-39) corrects fasting hypoglycemia in SUR-1-/- mice by lowering cAMP in pancreatic beta-cells and inhibiting secretion. J Biol Chem. 2008 Sep 19;283(38):25786-93. Siqi Wang, Yuqiong Wang , Long Lin, Zongjie Li, Fengyi Liu, Long Zhu, Jie Chen, Nianrong Zhang, Xinyu Cao, Sunman Ran, Genzheng Liu, Peng Gao, Weiliang Sun, Liang Peng, Jian Zhuang, and Hua Meng. Layer-Specific BTX-A Delivery to the Gastric Muscularis Achieves Effective Weight Control and Metabolic Improvement. Adv Sci (Weinh). 2023 Oct; 10(28): 2300822.
VU 065099 (25): Discovery of a novel series of orally bioavailable and CNS penetrant GLP-1R noncompetitive antagonists based on a 1,3-disubstituted-7-aryl-5,5-bis(trifluoromethyl)-5,8-dihydropyrimido[4,5-d]pyrimidine-2,4(1H,3H)-dione core. Nance et al. J.Med.Chem., 2017;60:1611.
GLP-1 (9-36) amide (29): Lotte Bjerre Knudsen, Lone Pridal. Glucagon-like peptide-1-(9-36) amide is a major metabolite of glucagon-like peptide-1-(7-36) amide after in vivo administration to dogs, and it acts as an antagonist on the pancreatic receptor. European Journal of Pharmacology 318 (1996)429-435.

Functional fragments and derivatives of such compounds may also be used, as far as they retain the biological activity of being GLP-1R antagonists.

In an embodiment, the GLP-1R antagonist is a monoclonal antibody against GLP-1R. For example, an antibody as disclosed in (26).

For "GLP-1R inhibitor" is intended a compound able to inhibit, decrease, alter, and/or modulate expression, function, and/or activity of GLP-1R, for example a compound able to inhibit the expression of a gene coding for GLP-1R.

Preferably, said inhibitor is a nucleic acid molecule preferably selected from: a single-stranded or double-stranded nucleic acid molecule, a miRNA molecule, a siRNA molecule, a shRNA molecule, an antisense oligonucleotide (ASO) (e.g. an antagomir, a Locked Nucleic Acid (LNA) modified ASO (LNA-ASO)), derivatives and mixtures thereof.

Preferably, said inhibitor has sufficient complementarity to at least a portion of the gene coding for GLP-1R or to its pre-mRNA or to its mRNA, to form a hybrid under physiological conditions.

Sequences of the gene coding for GLP-1R, of its pre-mRNA and of its mRNA are known in the art, see for example NCBI Reference sequence NM_002062.5 (Ensembl:ENSG00000112164), NCBI Release version of December 2023.

Said inhibitor can also be a vector, preferably a recombinant expression vector, comprising a coding sequence for the inhibitor as above defined and/or expressing the inhibitor as above defined, preferably under the control of a suitable promoter. Preferably, the vector is a viral or non-viral vector, more preferably the viral vector is selected from adeno-associated virus (AAV) vectors of any capsid serotype, either natural or artificial, lentivirus vectors, adenoviral vector, retroviral vectors, alphaviral vectors, vaccinia virus vectors, herpes simplex virus (HSV) vectors, rabies virus vectors, and Sindbis virus vectors.

In some embodiments, said inhibitor can be an interfering RNA, including but not limited to a small interfering RNA ("siRNA") or a small hairpin RNA ("shRNA"). Methods for constructing interfering RNAs are well known in the art. For example, the interfering RNA can be assembled from two separate oligonucleotides, where one strand is the sense strand and the other is the antisense strand, wherein the antisense and sense strands are self-complementary (i.e., each strand comprises nucleotide sequence that is complementary to nucleotide sequence in the other strand; such as where the antisense strand and sense strand form a duplex or double stranded structure); the antisense strand comprises nucleotide sequence that is complementary to a nucleotide sequence in a target nucleic acid molecule or a portion thereof (i.e., an undesired gene) and the sense strand comprises nucleotide sequence corresponding to the target nucleic acid sequence or a portion thereof. Alternatively, interfering RNA is assembled from a single oligonucleotide, where the self-complementary sense and antisense regions are linked by means of nucleic acid based or non-nucleic acid-based linker(s). The interfering RNA can be a polynucleotide with a duplex, asymmetric duplex, hairpin or asymmetric hairpin secondary structure, having self-complementary sense and antisense regions, wherein the antisense region comprises a nucleotide sequence that is complementary to nucleotide sequence in a separate target nucleic acid molecule or a portion thereof and the sense region having nucleotide sequence corresponding to the target nucleic acid sequence or a portion thereof. The interfering can be a circular single-stranded polynucleotide having two or more loop structures and a stem comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof, and wherein the circular polynucleotide can be processed either in vivo or in vitro to generate an active siRNA molecule capable of mediating RNA interference.

In some embodiments, the interfering RNA coding region encodes a self-complementary RNA molecule having a sense region, an antisense region and a loop region. Such an RNA molecule, when expressed desirably forms a "hairpin" structure and is referred to herein as an "shRNA." The loop region is generally between about 2 and about 10 nucleotides in length. In some embodiments, the loop region is from about 6 to about 9 nucleotides in length. In some embodiments, the sense region and the antisense region are between about 15 and about 20 nucleotides in length. Following post-transcriptional processing, the small hairpin RNA is converted into a siRNA by a cleavage event mediated by the enzyme Dicer, which is a member of the RNase III family. The siRNA is then capable of inhibiting the expression of a gene with which it shares homology. The terms "miRNA", "miR" and "microRNA" are used interchangeably and refer to a microRNA molecule found in eukaryotes that is involved in RNA-based gene regulation. Such terms refer to the single-stranded RNA molecule processed from a precursor. In some aspects, the term "antisense oligomers" can also be used to describe the microRNA molecules of the present disclosure. MicroRNAs recognize and bind to target mRNAs through imperfect base pairing leading to destabilization or translational inhibition of the target mRNA and thereby downregulate target gene expression. In animal cells, repression of gene expression by microRNAs is achieved by base-pairing to partially complementary sequences present mostly in 3' UTRs of target messenger RNAs (mRNAs), resulting in translation repression, mRNA degradation, or both. The microRNA seed sequence, essential for the binding of the microRNA to the mRNA, is a conserved sequence, which is generally situated at positions 2-8 from the microRNA 5'- end. MicroRNAs are genome-encoded sequences generally transcribed by RNA polymerase II into primary microRNAs (pri-microRNAs). The pri-microRNAs are then sequentially processed by two endonucleases of the RNAse III family: in the nucleus, Drosha processes the pri-microRNA into a precursor microRNA (pre-microRNA) of approximately 60-80 nucleotides, after which the pre-microRNA is further processed, in the cytoplasm, by Dicer to form a duplex containing two strands of about 19-23 nucleotides. The microRNA duplex is then unwound, and the mature microRNA is incorporated into the RNA-induced silencing complex (RISC), containing, among others, Argonaute and GW182 proteins essential for the silencing by microRNAs.

According to the present invention miRNAs or antisense oligonucleotides targeting the mRNA of the GLP-1R gene can be used as GLP-1R inhibitors.

The inhibitor described herein can be administered to the subject, for example, as nucleic acid without a delivery vehicle, in combination with a delivery reagent. In some embodiments, any nucleic acid delivery method known in the art can be used in the methods described herein.

In some embodiments, said inhibitor is a vector targeting GLP-1R gene to be administered together with a vector expressing a nuclease in order to knockdown GLP-1R using a CRISPR/Cas9 system.

In this embodiment, said vector targeting GLP-1R gene comprises at least one sequence complementary to the sequence of the GLP-1R gene, together with other suitable elements, as known in the art. The vector expressing a nuclease may comprise a nucleic acid coding for a CRISPR nuclease, such as Cas9 or spCas9 or SaCas9 preferably under the control of a tissue specific promoter, e.g. a liver specific promoter like a liver hybrid liver promoter (HLP). Said construct may further comprise a poly A, conveniently a short syntethic polyA (sh polyA). All such elements are well known in the art and may have conventional nucleotide sequences.

In some embodiments, the GLP-1R antagonist or inhibitor is for use for improving the effect of a cancer treatment in a subject in need thereof. In said embodiment, the GLP-1R antagonist can be administered before, simultaneously or after a further cancer treatment. Said cancer treatment can be chemotherapy, radiotherapy, immunotherapy or combinations thereof. Said cancer treatment is in particular a treatment with an immune checkpoint inhibitor, such as an antibody anti-PD-1, anti-PDL-1 or anti-CTLA-4.

In some embodiments, the GLP-1R antagonist or inhibitor is for use according to the invention in a subject who already underwent a cancer treatment, in particular a treatment with an immune checkpoint inhibitor, such as an antibody anti-PD-1, anti-PDL-1 or anti-CTLA-4.

It is also an object of the invention a nucleic acid coding for a GLP-1R antagonist or a vector expressing said nucleic acid for use for the prevention and/or treatment of a cancer.

In another aspect, the invention provides compositions, e.g., pharmaceutical compositions, for use for the prevention and/or treatment of a cancer which include a pharmaceutically acceptable carrier, excipient and/or stabilizer, and at least one GLP-1R antagonist or inhibitor, such as the ones described herein. In one embodiment, the composition, e.g., the pharmaceutical composition, includes a combination of the GLP-1R antagonist or inhibitor and one or more therapeutic agents, e.g., an anti-cancer therapeutic agent. Preferably, said further therapeutic agent is an active ingredient which is known to be effective in the prevention and/or treatment of a cancer, such as a IDO/TDO (Indoleamine 2, 3- dioxygenase/ tryptophan 2,3-dioxygenase) inhibitor, such as Epacadostat, an anti-EGFR (epidermal growth factor receptor) such as cetuximab, a platinum-based chemotherapy, e.g. cisplatin, carboplatin, or oxaliplatin, Paclitaxel/Carboplatin, a MEK (Mitogen-activated protein kinase kinase) inhibitor such as cobimetinib or regorafenib, a ERBB2 (v-erb-b2 avian erythroblastic leukemia viral oncogene homolog 2)-targeted antibody drug conjugate such as trastuzumab emtansine, Ipilimumab (BMS-734016), an anti-CTLA-4 (cytotoxic T-lymphocyte-associated antigen 4) such as Yervoy or Tremelimumab, an anti-PD-L1 such as durvalumab or atezolizumab (anti-PD-L1 (Programmed Death Ligand 1)), or an anti-PD-1(Programmed Death 1) such as nivolumab.

The GLP-1R antagonist or inhibitor can be administered to the subject in various embodiments in a formulation comprising suitable carriers, excipients, and other agents to provide improved transfer, delivery, tolerance, and the like, and suitable for an intravenous or subcutaneous injection.

The injectable preparations may be prepared by methods publicly known. For example, injectable preparations may be prepared, e.g., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 20 or 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injectable preparation thus prepared can be filled in an appropriate ampoule.

The GLP-1R antagonist or inhibitor for the use of the invention can be administered to the subject using any acceptable device or mechanism. For example, the administration can be accomplished using a syringe and needle or with a reusable pen and/or autoinjector delivery device.

According to the invention, "subject" means a human subject or human patient. In one embodiment, the subject is in need of inhibiting, reducing, neutralizing or blocking a cancer. In one embodiment, the subject has, or is at risk of having, a cancer.

The content of the GLP-1R antagonist or inhibitor in the pharmaceutical composition for the use of the invention is not limited as far as it is useful for treatment or prevention of a cancer, but preferably contains 0.0000001-10% by weight per total composition. The choice of the carrier may depend upon the route of administration and concentration of the active agent(s) and the carrier may be in the form of a lyophilised composition or an aqueous solution. Generally, an appropriate amount of a pharmaceutically acceptable salt is used in the carrier to render the composition isotonic. Examples of the carrier include but are not limited to saline, Ringer's solution and dextrose solution. Preferably, acceptable excipients, carriers, or stabilizers are non-toxic at the dosages and concentrations employed, including buffers such as citrate, phosphate, and other organic acids; salt-forming counter- ions, e.g. sodium and potassium; low molecular weight (> 10 amino acid residues) polypeptides; proteins, e.g. serum albumin, or gelatine; hydrophilic polymers, e.g. polyvinylpyrrolidone; amino acids such as histidine, glutamine, lysine, asparagine, arginine, or glycine; carbohydrates including glucose, mannose, or dextrins; monosaccharides; disaccharides; other sugars, e.g. sucrose, mannitol, trehalose or sorbitol; chelating agents, e.g. EDTA; non-ionic surfactants, e.g. Tween, Pluronics or polyethylene glycol; antioxidants including methionine, ascorbic acid and tocopherol; and/or preservatives, e.g. octadecyldimethylbenzyl ammonium chloride; hexamethonium chloride; benzalkonium chloride, benzethonium chloride; phenol, butyl or benzyl alcohol; alkyl parabens, e.g. methyl or propyl paraben; catechol; resorcinol; cyclohexanol; 3-pentanol; and m-cresol). Suitable carriers and their formulations are described in greater detail in Remington's Pharmaceutical Sciences, 17th ed., 1985, Mack Publishing Co. The composition may also contain at least one further active compound, such as a chemotherapeutic agent.

Preferably, the GLP-1R antagonist is included in a pharmaceutical composition for the use of the invention in an effective amount. The term "effective amount" refers to an amount sufficient to induce a detectable therapeutic response in the subject to which the pharmaceutical composition is to be administered.

### EXAMPLES

### Material and methods

### Human studies

Human peripheral blood mononuclear cells (PBMCs) were isolated from healthy donors' blood by Lymphoprep density gradient medium (Stem Cell Technology, Vancouver, BC-Canada). Subsequently, lymphocytes were extracted by apposite CD4 or CD8 mAb-coated microbeads (Miltenyi Biotec, Bergisch Gladbach - Germany). Study protocol was approved by Boston Children's Hospital IRB.

### Patients' characteristics

Human renal biopsies obtained from patients experiencing allograft rejection were obtained from Nephrology, dialysis and renal transplantation, Fondazione IRCCS Ca' Granda Ospedale Maggiore Policlinico Hospital, all patients' characteristics as well as their immunosuppressive regimen are described in Table 1. As regarding cardiac samples used in this study, samples were obtained from the right side of the interventricular septum of cardiac transplant recipients at De Gasperis Cardio Center and Transplant Center, Niguarda Hospital, Milan, Italy. Samples were then formalin-fixed, paraffin-embedded, sectioned, and histologically graded by a cardiac pathologist according to the International Society for Heart and Lung Transplantation criteria as previously reported (30). The Additional characteristics of patients and the immunosuppressive regimen are depicted in Table 2. As far regarding the lung samples used in this study obtained from Pulmonary Division, Boston Children's Hospital, the cases were blinded samples, with chronic lung allograft rejection, where evidences of oblierative broncholitis and the presence of lymphocytic bronchitis, epithelial cell injury and necrosis, have been confirmed.

### PBMCs isolation

Blood samples were obtained from healthy controls in accordance with Institutional Review Board committee approval. Peripheral blood mononuclear cell fractions were isolated by Ficoll density gradient centrifugation, and either processed for further analysis for FACS staining or subjected to cell isolation and enrichments for CD4⁺ and CD8⁺ T cells as previously described (31).

### Human flow cytometric analysis

To assess GPL-1R expression on human T cell subsets, peripheral blood mononuclear cell fractions isolated from healthy subjects were stained. The following antibodies were used for flow cytometric analysis in human studies: FITC-conjugated anti-human GLP-1R, BUV395-conjugated anti-human CD27, BV510-conjugated anti-human CD45RA, BV711-conjugated anti-human CCR7, BB700-conjugated anti-human CD4, BV605-conjugated anti-human-CD8, BV421-conjugated anti-human CD25, and PE-conjugated anti-human FoxP3. Antibodies were purchased from BD Biosciences, Alomone (Israel). FITC-conjugated donkey anti-rabbit IgG was used as related isotype controls. A BD Celesta flow cytometer (BD Biosciences) was used to analyze cells with the light scatter properties of stem cells or lymphocytes. Background staining was determined using nonreactive isotype-matched control mAbs with gates positioned to exclude 99% of nonreactive cells. FlowJo software version 10.6.2 (BD Bioscience) was used for analysis.

### Immunohistochemistry staining

Immunohistochemistry was performed with 4-pm-thick formalin-fixed, paraffin-embedded tissue sections. Photomicrographs (400X) were obtained using an Olympus BX41 microscope (Olympus, Center Valley, PA). The following primary antibodies were used: anti-GLP-1R, anti-CD3 (respectively from Alomone [Israel], Abcam [Cambridge, MA, and BD Biosciences [San Jose, CA]).

### Immunohistochemistry staining on kidney, lung and heart biopsies

The renal biopsies from transplant patients were paraffin-embedded following hospital standard protocol: paraformaldehyde (PFA) 4% for 30', dH2O for 5', ethanol (ETOH) 95% (10'x 3times), ETOH 100% (10'x 3times), Xylene (10'x 3times). Three different renal biopsies from transplant patients of 3 µm thick were evaluated for Haematoxylin Eosin (H&E Bioptica, Milan, Italy), CD3 (mouse anti-human Dako 1:20), and GLP-1R (rabbit Alomone Labs, Israel, 1:100) with the following final diagnosis f: i) Renal biopsies without major histological abnormalities, ii) Renal biopsies showing antibody-mediated rejection, ii) Renal biopsies showing T cell-mediated rejection. The hematoxylin & eosin was performed using a standard protocol and histochemistry was performed using super picture HRP polymer conjugated broad spectrum (Invitrogen, CA, United States). Images were acquired by AxioVision software 4.8 (Carl Zeiss). The IMAGE J program was used for the CD3 and GLP-1R quantification (n=18 region of interest).

### Murine studies

C57BL/6 and BALB/c mice were obtained from the Jackson Laboratory (Bar Harbor, Maine). Experiment with GLP-1R KO mice, were performed in Wuhan at the J. Yang Lab and all procedures involving these animals were performed and monitored in accordance with the guidelines of Tongji Animal Use Regulations and approved by the Institutional Animal Care and Use Committee of Tongji Medical College. All mice were cared for and used in accordance with Harvard Medical School institutional guidelines and housed 2 weeks in Boston Children Hospital pathogen-free animal house before undergoing experiments. Spleen, heart and renal cells were obtained by collagenase tissue digestion as previously described (32). Lymphocytes were extracted from murine spleen by apposite CD4 or CD8 mAb-coated microbeads (Miltenyi).

### Murine islet allotransplantation and interventional studies

Pancreatic islets from BALB/c mice were isolated by collagenase digestion and density gradient separation as previously described (33, 34). Subsequently, four to five hundred islets were inoculated under the renal capsule of each C57BL/6 recipient rendered diabetic with streptozotocin (STZ) (200 mg/kg, administered i.p. Sigma-Aldrich). Islet transplantation success was confirmed by glycaemia normalization. Rejection of islet allografts was defined as blood glucose levels >250 mg/dl for at least 2 consecutive days (35). Treatment modalities were the following: transplanted mice were intraperitoneally injected according to the following protocols: 1) Exendin-4 (Exe-4) (Amylin Pharmaceuticals, San Diego, CA -USA) 0.2pg twice/day for two weeks after transplant; 2) Exe-4 0.2pg twice/day from the day before until two weeks after transplant; 3) Exendin-4 2µg twice/day from the day before until two weeks after transplant; 4) Exendin-4 2µg twice/day + Exendin-9-39 (Exe-9) (Sigma-Aldrich) 2µg twice/day from the day before until two weeks after transplant; 5) Exendin-4 2µg twice/day from the day before until two weeks after transplant + Rapamycin 0.1mg/die for two weeks; 6) saline 0.5ml twice/day for two weeks. The follow-up of the transplanted mice was for 100 days.

### Murine cardiac allotransplantation and interventional studies

Cardiac allografts from BALB/c mice were transplanted intra-abdominally in C57BL/6 mice using microsurgical techniques as described (31). Rejection was determined as complete cessation of cardiac contractility and was confirmed by direct visualization. Transplanted mice were intraperitoneally injected according to the following protocols: 1) Exendin-4 (Exe-4) (Amylin Pharmaceuticals, San Diego, CA - USA) 0.2pg twice/day for two weeks after transplant; 2) Exe-4 0.2pg twice/day from the day before until two weeks after transplant; 3) Exe-4 2µg twice/day from the day before until two weeks after transplant; 4) Exe-4 2µg twice/day + Exendin-9-39 (Exe-9) (Sigma-Aldrich) 2µg twice/day from the day before until two weeks after transplant; 5) Exe-4 2µg twice/day from the day before until two weeks after transplant + Rapamycin 0.1mg/die for two weeks; 6) saline 0.5ml twice/day for two weeks.

### Colorectal tumour model

Female C57BL/6 mice were anesthetized with 100 mg/kg of ketamine and 50 mg/kg of xylazine. Next, by using a 29-gauge syringe, 2×10⁵ MC38 cells were injected submucosally into the distal posterior rectum in a final volume of 50 mL, as described previously (36, 37). MC38-injected mice were randomly assigned into 2 groups: (i) Exe-9-treated group (n=8), where all mice received an intraperitoneal injection of 2 µg of Exendin-9-39 (Exe-9) starting by Day1 for a total of 14 days; (ii) untreated-group (n=6), where all mice received an IP injection of PBS starting by day 1 and for a total of 14 days. Tumour growth was visually monitored daily. After animals' sacrifice, tumours were measured by a digital calliper measuring tumour length, width, and height and were weighted using a digital scale. Procedures involving mice wereb conformed to institutional guidelines in agreement with national and international law and were approved by the ethics committee by the Ministry of Health.

### Murine studies

### Adoptive transfer of CD3⁺ T cells from GLP-1R KO mice or from naïve C57BL/6 mice

2-4×10⁶ CD3⁺ T cells obtained from naive C57BL/6 mice and 2-4×10⁶ CD3⁺ T cells obtained from GLP-1R KO mice, were injected into BALB/c cardiac-transplanted C57BL/6 Rag1^{-/-} mice, and survival was assessed as previously described (32).

### Donor Specific Alloantibody Measurement

At day 7 post heart allotransplantation, we collected sera from all the recipients and determined the levels of donor-specific alloantibodies (DSA) using splenocytes from C57BL/6 donors as probes. In brief, splenic tissues were ground and filtered through a 70-µm cell strainer. The resulting splenocytes were then resuspended in PBS, seeded at 5×10⁵ per well into 96-well plates, incubated with an equal volume of fetal bovine serum (FBS) for 10 minutes to block nonspecific binding, washed with PBS containing 2% FBS, and then incubated with serum (1:8, 1:16 and 1:32 dilution) for 20 min at 4°C in the dark. Next, cells were washed twice and incubated for 20 minutes with PE-conjugated anti-mouse IgG (Southern Biotech, Birmingham, AL, USA; Cat# 1030-09).

The level of donor-specific IgG was then determined by FACS analysis and is presented as the mean fluorescence intensity (MFI).

### Treg induction assay

Naive CD4⁺ CD25⁻ T cells were selected and activated for 5 days with 1 µg/ml of plate-bound soluble anti-CD3 and anti-CD28 antibodies (BD Biosciences). Cells were cultured for 5-days in the presence of IL-2 (10ng/ml; R&D Systems) and TGFβ (2ng/mL; R&D Systems), anti-IL-4 (0.5µg/ml; PeproTech) and anti-IFNγ (2µg/mL; PeproTech) and were treated with Exe-4 (1nM) or left untreated. Then, cells were washed, pulsed for 4-hrs with phorbol myristate acetate (PMA; 50ng/mL; Sigma-Aldrich), ionomycin (500ng/mL; Sigma-Aldrich) and Golgi-stop (1.0µl/mL; BD Biosciences) and then analyzed by FACSCelesta (BD Biosciences) for the expression of Foxp3, Tbet and RoryT as previously reported (38).

### Heart tissue digestion and leukocyte isolation

Hearts were removed and washed with saline solution (PBS). For preparing single cell suspensions, heart tissue was minced with surgical scissors and incubated in 1 mg/ml collagenase type IV (Sigma-Aldrich, St. Louis, MO) in DMEM medium (Lonza, Walkersville, MD, USA) for 20 minutes at 37°C with gentle agitation. Cells were filtered through 70 µm filters and centrifuged (5 minutes at 4°C, 300 g). Cell pellets were resuspended in 33% Percoll solution (Sigma-Aldrich) and separated by gradient centrifugation by layering the cell suspension on top of a 66% Percoll solution before centrifugation for 20 minutes at 4°C. Cells at the interphase of the two solutions were carefully collected, washed in PBS and re-suspended in appropriate buffer for further experiments.

### Histology and immunohistochemistry

Immunohistochemistry was performed on formalin-fixed, paraffin-embedded tissue (23). Photomicrographs were taken using an Olympus BX41 microscope (Center Valley, PA - USA). Dr. Bernard Thorens kindly provided us with the anti-GLP-1R antibody for pathology studies. An expert pathologist evaluated selected micrographs.

### SDF-1 staining on islet allograft

Harvested islet allografts were fixed in PFA and paraffin-embedded. Tissues were cut into 5 µm sections, and respectively de-paraffinized, rehydrated and antigen-retrieved. Then, sections were incubated with the following antibody: mouse anti-CXCL12 monoclonal antibody (1:300, Thermofisher Scientific, MA5-23759). Images were acquired an Olympus BX41 microscope (Center Valley, PA) and analyzed by an expert pathologist.

### Flow cytometry

FACS analysis was performed with a FACSCelesta (BD Bioscience) on murine splenocytes or on single cells suspension prepared upon collagenase-digestion of grafted organs. We used the following antibodies: APC conjugated Rat anti-mouse CD62L, PE conjugated Rat anti-mouse CD44, PerCp conjugated Rat anti-mouse CD4 or PerCp conjugated Rat anti-mouse CD8, (Biolegend, San Diego, CA, USA), and APC conjugated anti-mouse/human insulin (R&D, Minneapolis, MN - USA). GLP-1R was detected by indirect staining; the primary antibody was purchased from Alomone Lab (Jerusalem, Israel) and variably associated with FITC or APC-conjugated anti-isotype Ab (BD and Biolegend respectively). For intracellular staining, splenocytes were isolated from BL6 mice were incubated with and w/o anti CD3/CD28 used both at 0.5 µg/ml, Exe-4 (0.1nM, 1nM and 10nM) or with Exe-4+Exe-9-39 (0.1nM, 1nM and 10nM). After 24hours incubation, cells were collected, washed and plated in RPMI 10% FBS, then stimulated with 50 ng/ml PMA (Sigma Aldrich), 500 ng/ml ionomycin (Sigma Aldrich) and Golgi Stop as recommended by the manufacturer (BD Biosciences, San Diego, CA, USA) for 4h in a humidified incubator 37°C, 5% CO₂. After incubation, cells were collected, washed and stained for surface markers, i.e CD4 BUV737, CD8 BUV395, (BD Biosciences, San Diego, CA, USA) and GLP-1R FITC (Alomone Lab, Jerusalem, Israel). They were further fixed, permeabilized using the BD Cytofix/Cytoperm Kit (BD Biosciences, San Diego, CA, USA), and stained with a mAb against IFN-γ (eBioscience). Finally, CD4⁺ GLP-1R⁺ IFN-γ⁺ and CD8⁺ GLP-1R⁺ IFN-γ ⁺ T cells were assessed by flow cytometry analysis. For the expression of GLP-1R on innate immune cells, the following antibodies were used: CD4 BB700, NK1.1 BV421, CD127 BV605, CD44 PE, GLP-1R FITC/PD1 FITC, Viability Dye BV510 (NKT subset); CD45 BV786, CD3 BB700, NK1.1 BV421, GLP-1R FITC/PD1 FITC, Viability Dye BV510 (NK subset); CD45 BB700, CD11c BV786, CD11b BV421, GLP-1R FITC/PD1 FITC, Viability Dye BV510; CD45 PE-CF594, CD127 BV605, NK1.1 BV421, CD335 BV786, CD49a BV650, CD117 BB700, IL33R PE, IL23R BV711, GLP-1R FITC/PD1 FITC, Viability Dye BV510 (LCs subset). For the determination of the chemokine receptors profile of GLP-1R⁺ CD3⁺ T cells, the following antibodies were used: GLP-1R FITC, CXCR4 PE, CX3CR1 BV605, CCR2 BV421, CCR5 BV650, CCR7 BV786, CXCR3 BB700, CXCR6 BV711, Viability Dye BV510.

### ELISpot assay

An ELISPOT assay was used to measure the number of IFN-γ-producing cells according to the manufacturer's protocol (BD Biosciences) as previously performed by our group (39). To test the role of GLP-1R-driven immunity on the anti-CD3/28 non-autoimmune specific response, 1×10⁶ splenocytes isolated from C57BL/6 mice were cultured in a microwell plate (BD Biosciences) coated with IFN-y capture antibody (Ab) in the presence of the following soluble anti-CD3 and anti-CD28 at 0,5ug/ml for 24 hours. Exe-4 and/or Exe-4+Exe-9-39 at concentrations of 0.1, 1 or 10 nM were added to the culture. After 24 hours, bound secondary Ab was visualized using HRP-streptavidin and the AEC substrate kit (BD Biosciences). Spots were counted using an Immunospot analyzer (Cellular Technology Ltd., Cleveland, OH) and expressed as the number of cytokine-producing spots per 1×10⁶ cells. After 24hours, ELISpot plates were developed and acquired on an ELISpot reader, as previously described.

### Western blot

Western Blot was performed with protein lysates from human and murine islets or CD4 and CD8 cells purified with magnetic beads (Miltenyi) from murine spleen or human Protein concentration in cell lysates was measured using a Bradford assay (Bio-rad, Hercules, CA). After an SDS polyacrylamide gel electrophoresis, proteins were transferred to a nitrocellulose membrane and incubated with primary GLP-1R (Alomone) or GAPDH (Cell Signaling Technology, Danvers, MA-USA) detecting Ab. The secondary incubation was performed with HRP-conjugated anti-isotype Ab (Cell Signaling). Quantification of band size was performed using ImageJ software and was normalized for GAPDH expression.

### qRT-PCR

Total RNA was extracted from human or murine immune cells. RNA was purified by using RNeasy kit (Qiagen, Valencia, CA), then reverse-transcribed into cDNA using Superscript III (Invitrogen, Carlsbad, CA). Transcripts were amplified using a 7300 Real-Time PCR System and analyzed by the ΔCT method; GAPDH was used as the internal reference (40). Both GLP-1R and GAPDH primers were obtained from Applied Biosystems (Foster City, CA).

### Transcriptomic array of GLP-1R^{pos} and GLP-1R^{neg} CD4⁺/CD8⁺ T cells

Transcriptomic array was performed on samples the following samples of GLP-1R^{pos} CD4⁺, GLP-1 R^{neg} CD4⁺ T cells, GLP-1R^{pos} CD8⁺ and GLP-1R^{neg} CD8⁺ T cells isolated from splenocytes of C57BL/6 mice. 1 µg of RNA was reverse transcribed using a Quantinova Reverse Transcription Kit (Qiagen, Hilden, Germany) following the manufacturer instructions. The obtained cDNA of each sample was then analyzed by quantitative real time PCR analysis using a SYBR green-based Quantinova LNA murine custom panel (Qiagen) according to the manufacturer's instruction. Relative quantification of each gene in the panel was calculated by delta-Ct method using averaged levels of *ACTB, B2M, GAPDH, HSP90AB1* genes for data normalization. Volcano plot shows statistical significance log (P value) versus difference (fold change).

### Phosphoproteomic screen of CD3⁺ T cells

Isolated CD3⁺ T cells from splenocytes were treated with Exendin-4 (Exe-4) for 24hrs or left untreated. Total soluble proteins from both cell extracts, approximately 7×10⁶ cells per conditions were used, all steps were performed using the Phospho Explorer Antibody Array kit (Full Moon Biosystems, PEX100) as recommended by the manufacturer (41). Next, soluble extracted proteins were biotinylated and were next incubated with a mixture of Cy3-streptavidin-detection buffer and the array slides were incubated on a rotating shaker for 30 min at RT. After, several washing steps, the array slides were allowed to dry by centrifugation. Dry array slides were analyzed by a slide-based microarray scanner as recommended by the manufacturer.

### Immunofluorescence of GLP-1R on CD3⁺ T cells

Immunofluorescence analysis of isolated CD3⁺ T cells and anti-CD3/anti-CD28-treated CD3⁺ T cells, was performed using the following antibody rabbit anti-GLP-1R FITC (AGR021F, Alomone) at a 1:200 dilution. Analysis of immunofluorescence and collection of images were conducted using a confocal system (TCS SP5 laser scanning confocal, Leica) as previously described.

### Immunoelectron microscopy

For electron microscopy immunocytochemistry, the pellets of lymphocytes were fixed for 2hrs at 4°C in a mixture of 2% paraformaldehyde and 1.25% glutaraldehyde in 0.05M (pH 7.3) cacodylate buffer, post-fixed in 1% osmium tetroxyde and embedded in EMBed-812 embedding kit (Electron Microscopy Sciences, Hatfield, PA, UK). Thin sections were incubated with GLP-1R primary antibody (Alomone labs) diluted 1:10, then with colloidal gold conjugated anti-rabbit antibody diluted 1:50 (EY laboratories, San Mateo CA USA), and, after counterstaining with uranyl acetate and lead citrate, were examined with a TEM Jeol 1010 (Jeol, Tokyo, Japan), of Centro Grandi Attrezzature of Varese Insubria University.

### GLP-1R transfection of T cells

The mGLP-1R cDNA construct (Origene) was inserted into the pMY-IRES-GFP plasmid (Cell BioLabs, Inc., San Diego, CA). The Platinum-E Retroviral Packaging cell line was transfected with the pMY-IRES-GFP plasmid expressing GLP-1R using polybrene (Santa Cruz Biotechnology), and supernatant was collected. T cells were activated for 48 hours in the presence of soluble anti-CD3/CD28 at 0.5 µg/ml each in the presence of IL-2 (400 ng/ml) (R&D Systems), and viral supernatant was added. The upregulation of GLP-1R was assessed by FACS and western blot analysis.

### Transwell migration assays

Transwell migration assays were performed on GLP-1R^{pos} and GLP-1R^{neg} CD3⁺ cells or respectively untransduced-mock CD3⁺ T cells or pmY-GLP-1R transduced CD3⁺ T cells, freshly isolated or activated with anti-CD3/CD28 (0.5 µg /ml each) in the presence or not of Exe-4 (1 nM). Chemotaxis assessments via transwell migration assay were performed using polycarbonate membranes with 5 µm pores (Corning) in accordance with the manufacturer's instructions and as previously published (42). Cells were washed, re-suspended in migration media (RPMI), and added to the upper chamber of the plate, while migration media containing 0 or 50 ng/mL SDF-1 (R&D Systems) was added to the bottom chamber. Treated cells were incubated over night at 37°C to migrate through the polycarbonate membrane into the bottom chamber. Input cell counts were obtained by flow cytometry with a BD FACSCelesta (BD, Biosciences) as well as cell counts of migrated cells in the lower chambers. Migration percentage was calculated as the number of cells in the lower chamber (migrated) compared to total input cell count for three separate wells. Mean migration percentages and standard deviations (SD) were calculated across the different donor cell lots. Analysis of lower chambers without SDF-1 (negative gradients) was used to control for any nonspecific increase in chemotaxis.

### Bioenergetic analysis

Cellular Bioenergetics were analyzed using the Seahorse Extracellular Flux Analyzer Xfe96 (Agilent Technologies, Santa Clara, CA, United States), which simultaneously measures Oxygen Consumption Rate (OCR, pmolO₂/min), ExtraCellular Acidification Rate (ECAR, mpH/min) and Proton Efflux Rate (PER, pmolH⁺/min). For Seahorse experiments 150.000 cells either freshly isolated GLP-1R^{pos} or GLP-1R^{neg} CD3⁺ T cells or activated with soluble anti-CD3/CD28 at 0.5 µg/ml each in the presence or not of Exe-4 (1 nM); in another setting untransduced mock CD3⁺ T cells or pmY-GLP-1R CD3⁺ T cells already activated with soluble anti-CD3/CD28 at 0.5 µg/ml each in the presence or not of Exe-4 (1 nM). Cells were resuspended in 50 µL assay medium (Agilent #103576-100 RPMI Medium pH7.4, supplemented with 10mM glucose, 2mM glutamine and 1mM Na-pyruvate) were seeded in each CellTak-precoated well of an Agilent Xfe96 Pro well plate. The cell plate was centrifuged 2 times at 40 × g (zero braking) for 1 minute. After centrifugation, 150 µL warm assay medium was gently added to each well. After 45 minutes incubation at 37 °C in a no-CO₂ incubator, Seahorse assays were performed according to:
(i) Seahorse XF T Cell Metabolic Profiling Kit (Agilent #103772-100) protocol which includes three measurements under basal condition and after the injection of the ATP synthase inhibitor oligomycin A (1.5 µM), the electron transport chain (ETC) accelerator ionophore BAM15 (2.5 µM) and the ETC inhibitors mixture rotenone (0.5 µM) + antimycin A (0.5 µM);
(ii) Seahorse XF Glycolytic Rate assay Kit (Agilent protocol #103344-100) which includes three measurements under basal condition and after the injection of the mixture rotenone (0.5 µM) + antimycin A (0.5 µM) and the glycolysis inhibitor 2-deoxyglucose (2-DG).

Seahorse parameters were normalized to cell number in each well. To this purpose, at the end of the Seahorse analysis, cell nuclei were stained with Hoechst 33342 (1 µg/mL) for about 15 min, imaged and counted with Operetta CLS^{™} software Harmony, as previously described (43).

Bioenergetic parameters were calculated using the following formulas:
Basal Respiration = mitoOCR = OCR_{basal}-OCR_{rot/ant};
Maximal Respiration = OCR_{BAM15} - OCR_{rot/ant};
Spare respiratory capacity = OCR_{BAM15} - OCR_{basal};
ATP linked Respiration= OCR_{basal}-OCR_{oligo};
Proton Leak= OCR_{oligo}-OCR_{rot/ant};
Non-mitochondrial respiration = OCR_{rot/ant}
Basal glycolysis = PER basal - mitoPER
mitoPER=mitoOCR*CCF; CCF=0.61;
Compensatory glycolysis = PER_{rot/ant}

### scRNA seq of GLP-1R^{pos} and GLP-1R^{neg} CD3⁺ T cells

Live GLP-1R^{pos} and GLP-1R^{neg} CD3⁺T cells isolated from splenocytes of C57BL/6 mice were FACS sorted through a BD FACSAria Fusion and were then washed and recovered in PBS containing 0.04% BSA. Recovered cells were counted and a viability > 90% was verified and the 2 cell suspensions (GLP-1R^{pos} and GLP-1R^{neg} CD3⁺ T cells) were then concentrated at a concentration of 1000 cells/µl and were subsequently used for library preparation. scRNA seq libraries were prepared using "Chromium scRNA 3'NEXTGEM 3.1" (10x GENOMIX) at the Center for Omics Sciences (COSR), IRCCS San Raffaele Scientific Institute, Italy, following manufacturer's instructions. Prepared libraries were then sequenced through an Illumina NovaSeq6000 on a S1 flow cell at 100 cycles.

### Bioinformatic analysis

UMItool v.1.0.0 was used to map the raw reads to the mouse genome (vs mm10) and to obtain the gene expression level for each cell, annotated with Genecode, release M22. The counts were then imported in the R environment v.4.0.3 and analyzed with the Seurat package v.4.3.0 (https://satijalab.org/seurat/). The two samples were first analyzed individually, following the standard Seurat pipeline. Cells with a total number of detected genes < 200 or >5000 were filtered out, as well as cells with a relative count of mitochondrial genes > 0.1. Counts were then normalized with the Seurat global-scaling normalization method "LogNormalize" with a 10,000 scale factor. The FindVariableFeatures function was used to identify a subset of features that exhibit high cell-to-cell variation that were then used as input for the RunPCA function to perform the principal component analysis (PCA). Both the datasets were then scaled with the ScaleData function, regressing out the number of feature and the percentage of mitochondrial genes to obtain equal weight in downstream analysis. CellCycleScoring was employed to assess cell cycle scores on scaled data and the distribution of the variable genes resulted to be unaffected by the cell cycle phases. Clustering was carried out through the FindCluster function, using the original Louvain algorithm and considering 1-30 principal components. Resolutions equal to 0.5 and 0.2 for sample GLP-1R^{pos} and sample GLP-1R^{neg}, respectively, were considered. The integrated analysis was performed with the standard Seurat pipeline. The two single samples were combined selecting the integration anchors through the SelectlntegrationFeatures and FindlntegrationAnchors functions. The final integration step was then performed with the IntegrateData function. Data were scaled as described above and the new integrated matrix was employed for clustering and visualization with a 0.3 resolution.

### Identification of differentially expressed genes

The function FindAllMarkers was used to perform a Wilcoxon test and to determine the genes differentially expressed in each cluster compared to the rest of the cells (logfc.threshold = 0.25, min.pct = 0.25). This allowed to detect the top marker genes for each cluster.

### Annotation

Cells were first classified using the SingleR package, with the ImmGen database (Aran et al. 2019) (44). Then, a more detailed manually cured annotation was performed, considering the top marker genes for each cluster.

### mRNA GLP-1R due diligence assessment on T cells

In order to confirm the expression of GLP-1R on T cells, total RNA was extracted from CD4⁺ and CD8⁺ T cells isolated by positive selection with microbeads (CD8a⁺ T Cell Isolation Kit, mouse and LT34 for CD4 isolation; MiltenyiBiotech.com) and from Beta-TC-6 *cell* line (BTC6), a murine beta cell line initially derived from insulinoma and used here as a positive control for GLP-1R expression. RNA was extracted by using the Direct-zol RNA Kit (Zymo Research Irvin, CA, USA) following manufacturer's instructions and then quality checked and quantified by spectrophotometry. A total of 10 ng RNA from the aforementioned cells was retrotranscribed into cDNA with the Superscript III kit (Life Technologies) and *Glp-1r* expression was quantified by qPCR using PowerUp SYBR Green Master Mix 2X (Life Technologies) on a QuantStudio S6 Real-Time PCR System (Thermo Fisher Scientific). Specificity of the amplifications was assessed by melt curve analysis. Primers for murine *Glp-1r* amplification (Fw: 5'-TGCTATCGGCGTCAACTTTCT-3' (SEQ ID No.1); Rv: 5'-ATTCAAGTGCTCCAGCCTCC-3' (SEQ ID No.2)) were designed in-house by an online tool (https://www.primer3plus.com), to yield a 334 bp-long amplicon from *Glp-1r* mRNA. Murine *Gapdh* mRNA was amplified in parallel using specific primers (Fw: 5'-CCAGGGCTGCCATTTGCAGTGGCAAAGTGG-3' (SEQ ID No.3); Rv: 5'-CCTGGAAGATGGTGATGGGVTTCCCGTTGA-3' (SEQ ID No.4)) to be used as positive internal control. Reactions in which reverse transcriptase was omitted (-RT) were run in parallel to test for potential DNA contamination. PCR products were separated on a 0.8% agarose gel in TAE buffer. Bands corresponding to the putative *Glp-1r* amplicon (334 bp) were excised from the gel and DNA was extracted using the Gel DNA Recovery Kit (Zymo research, Irvin, CA, USA). Both strands of recovered DNA were then sequenced by the Sanger method with the same forward and reverse primers used for gene amplification. The obtained amplicon sequences were aligned with that of nucleotides 950-1296 of murine *Glp-1r* mRNA NCBI Reference sequence NM_021332.2 by the Clustal Omega Multiple Sequence Alignment tool (https://www.ebi.ac.uk/Tools/msa/clustalo).

### Statistical analysis

Data are expressed as mean ± SEM. Kaplan-Meier analysis was used for analysis of survival. When two groups were compared, a two-sided unpaired Student t test (for parametric data) or a Mann-Whitney test (for nonparametric data) was used. A P value < 0.05 (by two-tailed testing) was considered an indicator of statistical significance. Graphs were generated using GraphPad Prism 6.0 software (GraphPad Software, San Diego, CA).

### Results

### GLP-1R is expressed in murine T cells and it expands during TCR stimulation

Firstly, we broadly assessed the expression of GLP-1R on lymphocytes by a variety of techniques including flow cytometry, WB, qRT-PCR and histological analysis. Murine CD4⁺ and CD8⁺ cells obtained from C57BL/6 mice expressed GLP-1R at protein and mRNA level; in all experiments murine islets were used as control (Fig. 1A-G). Additionally, to confirm the specificity of the GLP-1R expression by T cells, we conducted a due diligence assessment of *Glp-1r* expression at the mRNA level on respectively CD4⁺, CD8⁺ T cells as compared to a mouse beta-cell line (BTC6 cells), used as a positive control (Fig. 1H and 1I, Fig. 9A and 9B). We first designed exon junction-spanning primers to amplify a 334 bp-long target amplicon of the murine *Glp-1r* gene from cDNAs obtained from respectively CD4⁺, CD8⁺ and BTC6 cells. The correct amplification of the expected target amplicon size (334 bp) was confirmed/visualized by both melt curve analysis and by agarose gel electrophoresis and subsequent Sanger sequencing, which further confirmed the identity and conformity of *Glp-1r* mRNA in all tested samples (CD4⁺, CD8⁺ T cells and BTC6 cells) (Fig. 1H and 1I). Then the alignment of the PCR end-point product sequences with nucleotides 950-1296 of the *Glp-1r* reference cDNA (NM_021332.2) further confirmed the expression of *Glp-1r* in murine CD4⁺ and CD8⁺ T cells (Fig. 9C). Nearly 5-6% of CD4⁺ and CD8⁺ T lymphocytes expressed GLP-1R on their surface, while control islets are 40-42% positive (Fig. 1A-D). GLP-1R expression on T lymphocytes was also confirmed by immunohistochemistry staining; indeed, GLP-1R expression was also identified in the thymus and as expected, in pancreatic islets (Fig. 1J1 and 1J2, 1K1 and 1K2). We then investigated whether the expression of GLP-1R varied across the different CD4⁺ and CD8⁺ subsets. While no GLP-1R expression was evident on CD4⁺ or CD8⁺ naive T cells (defined as CD44⁻ /CD62L⁺ cells) and it was scanty (less than 5%) on memory cells (defined as CD44^{hi}/CD62L^{hi} cells), nearly 10-13% of CD4⁺ and CD8⁺ effector T cells (identified as CD44⁺CD62L⁻ cells) and 14% of regulatory T cells (Tregs, defined as CD4⁺CD25⁺Foxp3⁺ cells) appeared to be GLP-1R^{pos} (Fig. 1L-O and Fig. 10A and 10B). Our data indicates the presence of a discrete GLP-1R^{pos} population mainly confined to CD4⁺ and CD8⁺ effector cells and regulatory T cells. Based on these results and in order to get further insight into the role of GLP-1R expression and function, we challenged CD3⁺ T cells isolated from naive C57BL/6 WT mice with anti-CD3/anti-CD28 mAbs and GLP-1R expression was determined in a time course manner after 24, 72 and 120 hours of stimulation (Fig. 1P and 1Q). A substantial increase in GLP-1R expression, peaking at 120 hours, was evident during anti-CD3/anti-CD28 stimulation (Fig. 1P and 1Q). Immunofluorescence confirmed the substantial upregulation of GLP-1R protein in stimulated CD3⁺ T cells as compared to baseline (Fig. 1R and 1S). Notably, upon T cell activation, GLP-1R increased in expression matches PD-1 upregulation (Fig. 10C-H). Finally, we investigated GLP-1R expression on several components of innate immunity such as CD11c⁺ cells, CD11b⁺ cells, NK cells, NKT cells, and ILCs (ILC1, ILC2 and ILC3). We noticed an expression of GLP-1R by mainly NKT cells, NK cells, CD11c⁺ cells, CD11b⁺ macrophages while other cell types showed a scanty expression, always with a similar pattern to what was observed for PD-1 expression (Fig. 11A-C).

### GLP-1R^{pos} T cells showed a unique functional profile

we characterized GLP-1R^{pos} CD3⁺ T cells and denoted that proliferating cells showed a significant increase in GLP-1R protein expression as compared to their counterparts non-proliferating CD3⁺ T cells (Fig. 2A and 2B). This is another example of similarity between GLP-1R and PD-1 expression (Fig. 10C-H), being PD-1 upregulated in proliferating T cells as well. Interestingly, GLP-1R^{neg} CD3⁺ T cells revealed an abundant GLP-1R intracellular expression, although to a lower extent than that observed from their counterparts GLP-1R^{pos} CD3⁺ T cells (Fig. 2 C). This may appear to be a reservoir for a rapid conversion of GLP-1R^{neg} CD3⁺ T cells into GLP-1R^{pos} CD3⁺ T cells (Fig. 2D1 and 2D2). To further characterize the properties of GLP-1R^{pos} CD3⁺ T cells, we analyzed migration ability, proliferation in response to proximal TCR stimulation and metabolism. All data confirmed that GLP-1R^{pos} CD3⁺ T cells have a specific profile resuming those of activated/exhausted T cells as compared to the GLP-1R^{neg} CD3⁺ T cells. Indeed, an increased proliferation, an increased apoptosis and an enhanced migratory ability in response to SDF-1 were all features observed in GLP-1R^{pos} CD3⁺ T cells as compared to their counterparts GLP-1R^{neg} (Fig. 2E-H). The high migratory capacity of GLP-1R^{pos} CD3⁺ T cells toward SDF-1 matched their chemokine receptor profile which is enriched in CXCR4 and CCR2 expression (Fig. 11D). Furthermore, a significantly lower percentage of IFN-γ⁺ cells was obtained within GLP-1R^{pos} CD3⁺ T cells during an anti-CD3/anti-CD28 stimulation assay as compared to those observed within GLP-1R^{neg} CD3⁺ T cells (Fig. 2I). We further analyzed type 2 cytokine/ Tregulatory cytokine, IL-4 and IL-10, showing higher expression in GLP-1R^{pos} CD3⁺ T cells as compared to their negative counterparts (Fig. 11E and 11F). Finally, bioenergetic profiling showed that while the oxygen consumption rate (OCR), an indicator of oxidative phosphorylation (OXPHOS), was similar between GLP-1R^{pos} and GLP-1 R^{neg} CD3⁺ T cells under baseline conditions (Fig. 2J and 2K), GLP-1R^{pos} CD3⁺ T cells showed a significantly reduced maximal respiration in response to the uncoupling agent, BAM15, as compared to GLP-1R^{neg} CD3⁺ T cells (Fig. 2J and 2K). Particularly in GLP-1R^{pos} CD3⁺ T cells, OCR values significantly decreased to rotenone (complex I inhibitor) and antimycin A (complex III inhibitors), while remained unchanged in response to oligomycin (ATP synthase inhibitor). The spare respiratory capacity (SRC) was significantly reduced in GLP-1R^{pos} CD3⁺ T cells in comparison to their GLP-1R^{neg} counterparts (Fig. 2K). Other parameters such as ATP-linked respiration, ATP production rate and basal glycolysis evaluated in GLP-1R^{pos} CD3⁺ T cells, appeared to be similar to those revealed by their GLP-1R^{neg} counterparts (Fig. 2K). To further confirm our data obtained with OCR measurements, we then assessed the glycolytic profile of GLP-1R^{pos} CD3⁺ T cells compared to their GLP-1R^{neg} counterparts during a glycolysis stress assay (Fig. 2L and 2M). Our data did not reveal any differences in the glycolytic bioenergetic parameters (basal glycolysis and compensatory glycolysis) of the two subpopulations (Fig. 2L and 2M). Altogether, these results suggest that there are significant metabolic differences between GLP-1R^{neg} and GLP-1R^{pos} CD3⁺ T cells, as the former are hypermetabolic. We then confirmed that during anti-CD3/anti-CD28 stimulation, proliferating CD4⁺ as well as CD8⁺ T cells showed a significant increase in GLP-1R protein expression as observed for the whole CD3⁺ cells population (Fig. 2N and 2O). Again, GLP-1R behavior in CD4/CD8 was very similar to what was observed for PD-1 (Fig. 10G and 10H). GLP-1R^{pos} CD4⁺ and CD8⁺ T cells showed a higher cell death and lower IFN-y expression as compared to their counterparts GLP-1R^{neg} T cells (Fig. 2P and 2Q). We next performed a transcriptomic analysis of FACS sorted splenic GLP-1R^{pos} CD4⁺/CD8⁺ T cells and compared to their counterparts GLP-1R^{neg} (Fig. 2R-U). As illustrated in the volcano plot shown in Fig. 2R, we identified significant differences in the expression of 25 genes (adjusted p<0.05). Notably, the top significantly upregulated genes on GLP-1R^{pos} CD4⁺T cells appeared to be associated with apoptosis (CASP3, CASP7, Bak1), immunoregulatory profiles (TGFB1, TNFSF10, LGALS3) (Fig. 2R). Additionally, our gene ontology analysis revealed that most of the differently expressed genes particularly those downregulated in GLP-1R^{pos} CD4⁺T cells belong to mitotic cell cycle G1/S phase transition, and mainly those related to cellular activation and cytokines production (Fig. 2S). Significantly upregulated genes were mostly associated with a state of anergy and apoptosis signaling pathways (Fig. 2S). Similarly, the transcriptomic analysis was carried on in GLP-1R^{pos} CD8⁺ T cells as compared to their counterparts GLP-1 R^{neg} CD8⁺ T cells (Fig. 2T and 2U). Importantly, genes associated with T cell activation and particularly Th1 pathway activation were found upregulated on GLP-1R^{pos} CD8⁺ T cells (STAT6, LAT), while those downregulated genes were associated with response to ROS (CFLAR, JUN) and to protein phosphorylation (DGKA, JAK1) (Fig. 2T and 2U). Altogether, our findings suggested that GLP-1R signaling may confer an anergic and exhausted state in activated T lymphocytes and unveil a potential interactor network involved in GLP-1R signaling into T cells.

### scRNAseq unveils a unique molecular profile of GLP-1R^{pos} T cells

To further analyze the differential profile of GLP-1R^{pos} and GLP-1R^{neg} CD3⁺ T cells, we performed a scRNA sequencing. Following the standard Seurat pipeline as described in material and methods, we analyzed 3507 cells coming from sample GLP-1R^{pos} and 13518 cells from sample GLP-1R^{neg}. The FindCluster function identified 13 clusters in sample GLP-1R^{pos} and 7 clusters in sample GLP-1 R^{neg} (Fig. 3A and 3B). Annotation with SingleR provided major insights into cell types composing each cluster (Fig. 12A and 4B), while manually curated annotation, based on the expression of top marker genes for each cluster revealed the presence of distinct T cell subpopulations (Fig. 3D and 3E). To better characterize the differences between the cell populations within the two samples, we also applied the Seurat standard pipeline to perform an integrated analysis, combining GLP-1R^{pos} and GLP-1R^{neg} datasets (Fig. 3C). A total of 17025 cells were considered, resulting in 11 clusters representing distinguished T cell subpopulations (Fig. 3F and 3G). Our data analysis suggested the presence of a heterogeneous T cell subpopulation within GLP-1R^{pos} CD3⁺ T cells, with the main dominating subset composed of CD8 exhausted T cells, as depicted in the UMAP and heatmap, illustrating the top 5 genes related to each cluster (Fig. 3D and Fig. 12C). In particular, the analysis uncovered highly expressed genes in clusters 0, 1, and 2, representing CD4 and CD8 T cells, which correlated with a T cell exhaustion state (dapl1, Ncl), other immunosuppressive genes (lef1, Dusp10), and various immunoregulatory genes (CTLA-4, Tnfrsf4, Dusp1, Ncl) among others (Fig. 12C). In contrast, the analysis of the GLP-1R^{neg} CD3⁺ T cell subpopulation highlighted the presence of a dominating subset composed of CD8 effector memory T cell (composing clusters 0 and 6), with higher expression of the Natural Killer cell granule protein 7 (Nkg7) gene, which confers potent synapse efficiency of tumor infiltrating cytotoxic CD8 T cells (Fig. 12D-I). Additionally, the 11 clusters identified in the integrated dataset were further inspected with manually cured annotation, revealing T cell subpopulations such as CD4, CD8 naive, memory cells, CD4 regulatory T cells and exhausted T cells (Fig. 3F and 3G). Overall, our scRNAseq analysis showed the existence of molecular and functional dissimilarities and heterogeneity between GLP-1R expressing cells and their negative counterparts, with the former being mainly composed of CD8 exhausted T cells and the latter being composed of CD8 cytotoxic T cells.

### GLP-1R signaling modulates T cells activation, anergy and apoptosis

In order to understand the molecular basis of GLP-1R signaling, we performed a phosphoproteomic arrays, with over 1500 of known proteins tested for expression and activation during a T cell *in vitro* challenge with GLP-1R agonist (Exenatide/Exendin-4). We found that GLP-1R signalling altered the expression and the activation of several signalling molecules into T cells, including the p38-MAPK-p53 pathway, already described to be involved in senescence and exhaustion (Fig. 4A and 4B). Strikingly, most of the identified activated pathways are related to TCR triggering signaling such as IL-2, IL-21 and AP-1 (Fig. 4B). Then, to further mechanistically explore T cell modulatory effects of GLP-1R signaling we performed a set of complimentary *in vitro* assays with the use of the GLP-1R agonist Exendin-4 (Exe-4) and of the GLP-1R antagonist Exendin-9-39 (Exe-9). First, we observed that GLP-1R signaling with Exe-4 significantly reduced the number of IFN-y producing cells by mitogen-stimulated murine splenocytes in an IFN-y ELISpot assay (Fig. 13A), while the addition of Exe-9 significantly hampered the overseen immunoregulatory effect of Exe-4. When, splenocytes were activated with anti-CD3/anti-CD28 alone a significant increase in a cell death with no effect on the proliferation of CD4⁺ and CD8⁺ T cells was evident upon challenge with Exe-4 alone, while the addition of Exe-9 reduced the effect (Fig. 13B-E). We next assessed the impact of signalling through GPL-1R on proliferation, apoptosis, migration, IFN-y production, and metabolism of GLP-1R^{pos} and GLP-1R^{neg} CD3⁺ T cells subpopulations stimulated with anti-CD3/anti-CD28 in the presence of Exe-4 (Fig. 4C-H). Exe-4-treatment slightly modulated the proliferation rate of GLP-1R^{pos}, but not of GLP-1R^{neg} CD3⁺ T cells (Fig. 4C). GLP-1R^{pos} CD3⁺ T cells appeared significantly more apoptotic as compared to their counterparts GLP-1R^{neg} CD3⁺ T cells, independently of Exe-4 treatment (Fig. 4D). While Exe-4-treatment reduced the migratory potential of GLP-1R^{neg} CD3⁺ T cells only, no evident effect was observed on IFN-y expression, with the GLP-1R^{pos} CD3⁺ T cells migrating less and retaining lower IFN-y expression (Fig. 4E and 4F). Notably, Exe-4 treatment slightly increased the mitochondrial respiration of GLP-1R^{pos} CD3⁺ T cells (Fig. 4G and 4H), as revealed by an increase in OCR values at all time points and in maximal OXPHOS, with no effect on mitochondrial ATP production (Fig. 4G and 4H, Fig. 13F). We further assessed the concentration of total GLP-1 in different assays, and we never detected the presence of GLP-1 (Fig. 13H). We then, wondered whether signalling through GPL-1R may influence Treg induction, function and stability and upon co-culture with Exe-4. Our data indicate no substantial differences upon addition of Exe-4 to naive CD4⁺CD25⁻ T cells during Treg induction assay (Fig. 13I and 13J). Additionally, we observed some effect on iTreg stability upon addition of Exe-4 with a significant reduction of Ror-yT and Tbet (Fig. 13K-N). Finally, when iTregs were generated in the presence of Exe-4, at a ratio of 1: 4 to effector T cells, a significantly reduced suppression was observed as compared to when untreated-iTregs were added at the same ratio (Fig. 13O-R).

### GLP-1R genetic gain of function and T cell properties

Next, we sought to determine the effect of GLP-1R genetic gain of function; indeed, GLP-1R cDNA was transduced by using a pRetroG-CMV-GLP-1R retroviral vector into CD3⁺ T cells (GLP-1Rtg CD3⁺ T cells), and upregulation of GLP-1R expression was confirmed by FACS (Fig. 14A) and by western blot analysis (Fig. 14B). Exe-4 treatment has no effect on the proliferation of both untransduced-mock and pmY-GLP-1R transduced CD3⁺ T cells (Fig. 4I), while a significantly increased apoptosis was observed in pmY-GLP-1R transduced CD3⁺ T cells only (Fig. 4J). Moreover, Exe-4 treatment reduced the migratory ability in response to SDF-1 in both untransduced-mock and pmY-GLP-1R transduced CD3⁺ T cells (Fig. 4K) without any effect on IFN-γ expression in both groups (Fig. 4L). Exe-4 treatment significantly increased OCR values and ATP production from mitochondrial respiration in pmY-GLP-1R transduced CD3⁺ T cells as compared to untransduced-mock CD3⁺ T cells (Fig. 4M and 4N and Fig. 13G).

### GLP-1R genetic loss of function and T cell properties

To extend our findings into an *in vivo* setting, we used a GLP-1R KO mice, in which the baseline phenotype was described (Fig. 14C) and the absence of GLP-1R was confirmed within several immune organs (Fig. 14D-P). We first performed an apoptosis assay on CD3⁺ T cells extracted from GLP-1R KO mice and stimulated with anti-CD3/CD28 as compared to those isolated from WT mice. A decline in CD3⁺ T cells apoptotic rate was seen when GLP-1R is genetically knocked down (Fig. 14Q-S). Next, we assessed IFN-γ expression by CD3⁺ T cells extracted from GLP-1R KO mice as comparatively to those observed in WT mice (Fig. 14T). A significant increase in the percentage of IFN-γ⁺CD3⁺ T cells from GLP-1R KO mice was observed at baseline and more profoundly upon anti-CD3/CD28 stimulation as compared to those of WT mice (Fig. 14T). We then tested the effect of lacking a viable GLP-1R signaling in an *in vivo* murine model of allograft tolerance where BALB/c hearts were intra-abdominally transplanted into C57BL/6 WT mice or into C57BL/6 GLP-1R KO mice. Both groups were treated with low dose CTLA4-Ig to induce long-term tolerance (Fig. 4O). While C57BL/6 WT mice transplanted with BALB/c hearts and treated with low dose CTLA4-Ig showed an extended survival of the allograft (MST=58 days) (Fig. 4O), an acceleration of heart allograft rejection was observed in C57BL/6 GLP-1R KO mice (MST=28 days) (Fig. 4O). Pathology of allograft showed a severe and accentuated infiltration of CD3⁺ and CD8⁺ T cells as revealed by hematoxylin and eosin, CD3 and CD8 staining (Fig. 4P-R, Fig. 14U1 and 14U2, 14V1 and 14V2, and 14W). Moreover, we observed an increase in the degree of fibrosis as evaluated with Masson's trichrome staining in the GLP-1R KO group (Fig. 4S-U). A substantial increase in CD4 effector T cells as well as significant increases in the percentages of IFN-γ⁺ CD4⁺ T cells and IL-17⁺ CD4⁺ T cells (Fig. 4V-X) and slightly in CD8 effector T cells (Fig. 14X) were evident in the KO mice. Importantly, significant increase in the percentage of IL-17⁺ CD8⁺ T cells but not of IFN-γ⁺CD8⁺ T cells, were observed in GLP-1R KO recipients as compared to WT recipients (Fig. 14Y and 14Z) with a marked decrease in CD4⁺ Tregs in the GLP-1R KO group (Fig. 4Y). Our results indicate that GLP-1R signaling might impact T cells compartment by favoring their apoptosis thus modulating the alloimmune response.

### GLP-IR signaling prolongs islet allograft survival

Then we investigated the role of GLP-1R and its signaling in an *in vivo* murine models of allotransplantation. STZ-treated C57BL/6 mice received an islet transplant from BALB/c donors and were left untreated. At day 0, GLP-1R expression was confirmed on splenic CD4⁺ and CD8⁺ T cells (Fig. 5A and 5B, Fig. 15A and 15B), while the graft was clear from infiltrating CD4⁺ and CD8⁺ T cells (Fig. 5C and 5D, Fig. 15C and 15D). At day 14, all mice displayed acute allograft rejection associated with an increase in GLP-1R^{pos} cells among both splenic CD4⁺ and CD8⁺ T cells (Fig. 5A and 5B, Fig. 15A and B) and islet allograft infiltrating CD4⁺ and CD8⁺ T cells (Fig. 5C and 5D, Fig. 15C and 15D). The presence of graft-infiltrating GLP-1R^{pos} T lymphocytes at day 14 was further confirmed by immunohistochemistry analysis (Fig. 5E1-E3 and 5F). Interestingly, the administration of low dose of the GLP-1R agonist (Exe-4) resulted in a prolongation of islet survival up to 100 days in 20% of the recipients ([MST]=41 days vs. untreated ([MST]=12 days), p=0.001), while 60% of those that received high doses of Exe-4 preceded by a pre-treatment showed a prolongation of islet survival up to 100 days ([MST]=67 days vs. untreated, p<0.001) (Fig. 5G). The co-administration of Exe-4 and rapamycin was associated with 100% of graft survival at the end of follow-up (day 100) (Fig. 5G). The beneficial effects of Exe-4 were partly abolished by the co-administration of its antagonist Exendin-9-39 (Exe-9) (Fig. 15E). Hematoxylin and eosin staining on islet allograft at day 14 revealed a decreased graft infiltration in Exe-4-treated mice, while the co-administration of Exe-9 restored the graft infiltration (Fig. 15F1-F3 and 15G). Exe-4 treatment induced a reduction of CD8⁺ but not of CD4⁺ effector memory cells and a significant increase of Treg lymphocytes, with Exe-9 co-administration restored CD8⁺ effector cell number but did not affect Tregs (Fig. 15H-J). Notably, SDF-1 immunostaining revealed a mild SDF-1 expression within islet allograft infiltrate matching CXCR4 expression by GLP-1R^{pos} cells (Fig. 15K1 and 15K2, Fig. 15L1 and 15L2). Taken together, our results confirmed that GLP-1R is upregulated on T cells during islet allograft rejection, that GLP-1R^{pos} T cells infiltrates the graft and that signaling through GLP-1R with an agonist has an important immunoregulatory effect.

### GLP-1R signaling prolongs heart allograft survival

Because of the wide range of effects that GLP-1R has on islets (24), that may confound the immunological effect, we next sought to confirm the islet-independent immunoregulation of GLP-1R signaling by using an MHC-fully mismatched model of heart transplantation. Briefly, C57BL/6 mice received a heart transplant from BALB/c donors and were left untreated. At day 7, all mice displayed acute allograft rejection associated with an increase in GLP-1R^{pos} cells among both splenic (Fig. 5H and 5I, Fig. 15M and 15N) and heart allograft infiltrating CD4⁺ and CD8⁺ T cells (Fig. 5J and 5K, Fig.15O and 15P). The presence of graft-infiltrating GLP-1R^{pos} T lymphocytes at day 7 was confirmed by immunohistochemistry analysis, while cardiomyocytes and otherparenchymal cells appeared negative for GLP-1R staining (Fig. 5L1-L3 and 5M). Moreover, while untreated recipients rejected cardiac allograft within a range of 7 days ([MST]=7 days), (Fig. 5N), Exe-4-treated recipients receiving either a low or a high dose of Exe-4 preceded or not with pre-treatment showed a significant delay in allograft rejection (respectively [MST]=9, 10 and 10 days; p<0.01 as compared to untreated) (Fig. 5N). Notably, transplanted-mice receiving a combination therapy with Exe-4 and rapamycin showed a significant improvement in graft survival with indefinite long-term cardiac transplant survival in almost 20% of recipients ([MST]=39 days, p<0.001 as compared to untreated), suggesting the existence of a synergistic effect of Exe-4 with rapamycin (Fig. 5N). The beneficial effects of Exe-4 were partly abolished by the co-administration of its antagonist Exe-9 (Fig. 15Q). Hematoxylin and eosin staining (Fig. 15R1-R3 and 15S) revealed a decrease of graft infiltration in Exe-4-treated mice that wasabolished by Exe-9 co-administration (Fig. 15R3 and 15S). Notably, a significant decrease of CD4⁺ and CD8⁺ effector T cells was revealed in Exe-4-treated mice that was reverted by Exe-9 co-administration (Fig. 15T and 15U), while no effect on Tregs in Exe-4-treated mice was observed (Fig. 15V). In a tentative to test the effect of GLP-1R on a more selective way, we performed a series of adoptive transfer studies of WT and GLP-1R KO T cells in a model of heterotopic intra-abdominal heart transplantation, where recipients C57BL/6 Rag1^{-/-} mice received a heart transplant from BALB/c. At the day of transplant, all recipients received an adoptive transfer of either splenic WT CD3⁺ T cells or of splenic GLP-1R KO CD3⁺ T cells in the latter accelerating allograft rejection in the Rag1^{-/-} group (Fig. 16A). Furthermore, genetic loss of function of GLP-1R did not affect alloantibody formation neither the levels of circulating follicular helper T cells (cTfh) in both groups (Fig. 16B and 16C). Heart transplantation data confirmed what is obtained in the islet model, and further suggested the relevance of GLP-1R signaling for T cell compartment.

### GLP-IR antagonism triggers an antitumor immunity

To investigate the hypothesis that GLP-1R may play a role as an immune check point, we studied the tumour response to a systemic administration of a GLP-1R antagonist, Exendin-9-39 (Exe-9) in an experimental murine model of colorectal cancer. In this model we injected MC38 colorectal cancer cells into the distal posterior rectum as described previously (37), a group of mice (n=8) were assigned to receive 2 µg of Exe-9 once a day for 14 days; another group of mice (n=6) were left untreated (Fig. 6A and 6B). Indeed, mice treated with Exe-9 showed a significantly reduced tumour size (Fig. 6C), and mesenteric lymph nodes volume (Fig. 6D), while no differences were observed comparing spleen weight and colon length in both groups (Fig. 6E and 6F). Pathological examination of the tumors in both groups revealed a significant intratumoral infiltration of CD3⁺ T cells within Exe-9-treated group (Fig. 6G-I). Additional mechanistic studies on the tumors from both groups further confirmed the increased infiltration of CD3⁺ T cells in the Exe-9-treated group (Fig. 6J) and of particularly activated effector memory CD8⁺ T cells (Fig. 6K-M). While no major differences were observed comparing CD3⁺ and CD8⁺ effector memory T cells within the spleen and mesenteric lymph nodes of both groups ( Fig. 6N-Q). Altogether, our data showed an evident proof-of-concept that GLP-1R antagonist may trigger T-cell-mediated anti-tumor activity.

### GLP-1R is relevant for human T cells

We next sought to explore the relevance of GLP-1R on human T cells as well. Our data confirmed the expression of GLP-1R on CD4⁺ and CD8⁺ T cells (Fig. 7A-D). Human PBMCs extracted from healthy individuals revealed at FACS analysis an average GLP-1R expression on CD4⁺ and CD8⁺ T cells of about respectively 6.1±1.2% and 28.3±4.1% (Fig. 7A-D), while human islets express GLP-1R at an average of 46.3.0±0.2% (Fig. 7C and 7D). GLP-1R expression on T cells was further confirmed by western-blot analysis at protein level (Fig. 7E and 7F) and at mRNA transcript with qRT-PCR analysis (Fig. 7G). Consistently with murine data, GLP-1R expression was found by immunohistochemistry in human thymus and pancreas (Fig. 7H1 and 7H2, 7I1 and 7I2). In situ hybridization confirmed the expression of GLP-1R mRNA in human thymus and spleen (Fig. 17A and 17B). Notably, GLP-1R expression was evident in human regulatory CD4⁺ T cells identified as CD4⁺CD25^{hi}CD127-FoxP3⁺ T cells (Fig. 17C and 17D), in effector memory CD4⁺ and CD8⁺ T cells (defined as CD4⁺CD45RA⁺CCR7⁻ or CD8⁺CD45RA⁺CCR7⁻ cells), (Fig. 7J-M). While central memory CD4 and CD8 T cells (denoted as CD4⁺CD45RA⁺CCR7⁺ or CD8⁺CD45RA⁺CCR7⁺ cells) appeared to express GLP-1R at a lower extent (Fig. 7J-M). Importantly, CD4⁺ / CD8⁺ terminally differentiated effector memory T cells appeared highly positive for GLP-1R (Fig. 7J-M). Paralleling our findings in murine studies, we sought to determine if GLP-1R plays a role during TCR mediated-stimulation, we assessed GLP-1R expression in a time course manner on stimulated Cell-trace violet labelled CD3⁺ T cells with soluble anti-CD3/anti-CD28 Abs (Fig. 7N and 7O). GLP-1R protein expression increased after 24 hours following TCR stimulation and peaked at 120 hours (Fig. 7N and 7O). Immunofluorescence confirmed our observation showing substantial increase in GLP-1R protein expression on CD3⁺ T cells (Fig. 7P and 7Q). Proliferating CD3⁺ T cells, analyzed with cell trace dilution upon TCR stimulation with soluble anti-CD3/CD28, appeared highly expressing GLP-1R as compared to their counterparts non-dividing/proliferating CD3⁺ T cells (Fig. 17E and 17F). The same observation was confirmed on dividing CD4⁺ T cells and CD8⁺ T cells where a significant increase in GLP-1R expression was evident as well (Fig. 17G and 17H). Taken together, these findings underlined the expression of GLP-1R in human T lymphocytes, mainly confined to effector memory CD4⁺ and CD8⁺ T cells and a substantial upregulation of GLP-1R on T cells upon proximal TCR stimulation, thus paralleling the murine data.

### Expansion of GLP-1R^{pos} T cells during human allograft rejection

Next, we posit to explore the relevance and the presence of GLP-1R in a series of graft rejecting biopsies obtained from individuals recipients of kidney, heart or lung allografts (Fig. 8, Tables 1 and 2). We first evaluated the expression of GLP-1R in human renal biopsies obtained from patients experiencing allograft rejection. Pathology showed an active T-cell mediated rejection, with areas of interstitial fibrosis and tubular atrophy associated with moderate or severe tubulitis. Significant GLP-1R^{pos} CD3⁺ T cells intragraft infiltration was observed, while no evidence of GLP-1R^{pos} CD3⁺ T cells were found in control biopsies obtained from non-rejecting renal allografts (Fig. 8A1-A3, Fig. 8B1-B3, Fig. 17I and 17J). Importantly, we evaluated cases of antibody-mediated kidney rejection, and we did not report any significant GLP-1R expression (Fig. 17K1-K3 and 17L1-L3), confirming thus that the overexpression of GLP-1R is specific for T cell-mediated rejection. We then confirmed the relevance of GLP-1R^{pos} CD3⁺ T cells in heart and lung allograft rejection. Particularly, GLP-1R^{pos} CD3⁺ T cells were observed in biopsies obtained from cardiac-transplanted patients, with and without features of cardiac allograft rejection and vasculopathy (Fig. 8C1-C3 and Fig. 8D1-D3). Additionally, we reported the presence of intragraft GLP-1R^{pos} CD3⁺ T cells in lung biopsies obtained from cases with chronic lung allograft rejection, where evidences of oblierative broncholitis and the presence of lymphocytic bronchitis, epithelial cell injury and necrosis, have been confirmed (Fig. 8E1-E3 and Fig. 8F1-F3). Taken together, our clinical finding further reinforced and confirmed the relevance of GLP-1R in clinical allograft rejection.

### Discussion

Recent evidence suggested that GLP-1R may have a role in immune response (45); indeed the use of several GLP-1R agonists (Exenatide, Liraglutide) offered a substantial benefits in a wide range of inflammatory diseases (14, 16, 46, 47). Several reports investigated the anti-inflammatory proprieties of GLP-1R: several studies focused on macrophages (48) and some on T lymphocytes (11, 13, 16, 49-51). The importance of GLP-1R in limiting gut intraepithelial lymphocyte related-inflammation has been reportedly described by suppressing their effector function and dampening TCR proximal signaling in a PKA-dependent manner (52). *In vivo,* Exenatide was able to delay diabetes onset and to prolong islet transplantation in NOD mice (15, 16). Inventors have stated that they detected a discrete population of GLP-1R^{pos} cells, mainly located within CD4⁺ and CD8⁺ effector memory T cells, which increased substantially after alloimmune response. Interestingly, they also showed that the use of GLP-1R agonist modulate the T cell alloimmune response and preserve allograft survival. They further posited that the presence of GLP-1R on T cells is associated with a shift toward an anergic profile, as confirmed by the substantial expression of exhaustion and anergy related genes, which may support the hypothesis that GLP-1R plays a role of negative costimulatory molecule during immune response. Inventors' main data showed that GLP-1R expression is limited to CD4 and CD8 effector-memory and regulatory T cells and not detectable in central-memory and naive subsets. Furthermore, they showed that a population of terminally differentiated effector memory CD4⁺ and CD8⁺ T cells (i.e.; EMRA T cells) expresses GLP-1R at highest levels; indeed, terminally differentiated effector memory T cells are described in the literature as having high tendency to apoptosis, cytotoxic activity and their active role in allotransplant rejection has been established (53, 54). Importantly, they showed that GLP-1R^{pos} CD4 and CD8 cells infiltrate the graft at high percentage and are expanded in the spleen of transplanted mice, while the treatment with GLP-1R agonist was able to prevent heart and islet graft infiltration and to prolong graft survival. Pharmacological and genetic modulation of GLP-1R all confirm that the signaling through the receptor is immunoregulatory, while the absence of GLP-1R accelerate the rejection in chronic heart transplant model of CTLA4-Ig-induced tolerance.

Finally, an important finding of the inventors is that GLP-1R antagonism with Exendin-9-39 applied into a relevant murine cancer model *in vivo,* was able to trigger an anti-tumor immune response.

In conclusion, inventors demonstrated that GLP-1R receptor is expressed by a variety of T cell subsets and it has a role in controlling T cell activation, IFN-y production, cell metabolism and death thus acting as a negative costimulatory molecule. GLP-1R^{pos} T lymphocytes are expanded during the alloimmune response and GLP-1R signaling strives a T cell negative costimulatory signal mimicking those shown by PD-1.

### Tables

**Table 1. Characteristics of kidney-transplanted patients. Footsteps: AMR - Antibody Mediated Rejection, TCMR - T-Cell Mediated Rejection, sCr - Serum Creatinine, eGFR - estimated Glomerular Filtration Rate, HbA1C - Glycated Hemoglobin, WBC - White Blood Cells, N% - Percentage of Neutrophils, L% - Percentage of Leukocytes, Hb - Hemoglobin, CRP - C-Reactive Protein, DSA - Donor Specific Antibodies, MH - Microhematuria, MFI - Mean Fluorescent Intensity**

| | Normal | | | AMR | | | TCMR | | |
|---|---|---|---|---|---|---|---|---|---|
| | 1 | 2 | 3 | 1 | 2 | 3 | 1 | 2 | 3 |
| **General Characteristics** | | | | | | | | | |
| Age (years) | 29 | 52 | 31 | 48 | 69 | 60 | 53 | 35 | 70 |
| Transplant Age (years) | 1,3 | 0,1 | 2,2 | 13,9 | 10,3 | 9,2 | 1,2 | 0,2 | 0,4 |
| Sex (M / F) | F | F | M | F | M | M | M | F | F |
| Hypertension (Yes / No) | Yes | No | No | Yes | No | Yes | No | Yes | No |
| Diabetes mellitus (Yes / No) | No | No | No | No | Yes | Yes | No | No | Yes |

| **Biochemical Characteristics** | | | | | | | | | |
|---|---|---|---|---|---|---|---|---|---|
| sCr (mg/dl) | 2 | 1,54 | 2,36 | 1,91 | 1,24 | 1,29 | 1,7 | 2,97 | 1,4 |
| eGFR (ml/min/1.73 m²) | 33 | 38,4 | 35,4 | 30,4 | 58,9 | 59,9 | 45 | 19,6 | 38 |
| Urea (mmol/l) | 95 | 57 | 51 | 90 | 67 | 39 | 56 | 109 | 54 |
| Glucose (mg/dl) | 81 | 86 | 82 | 118 | 161 | 187 | 83 | 77 | 128 |
| HbA1C (mmol/mol, %) | 40 (5,8) | 35 (5,3) | 38 (5,6) | 31 (5) | 47 (6,5) | 64 (8) | 45 (6,3) | 42 (6%) | 53 (7) |
| WBC (cells/ul) | 4950 | 7300 | 8600 | 6700 | 3300 | 10000 | 6400 | 7900 | 5500 |
| N% / L% | 40 / 48 | 89 / 9 | 25 / 68 | 57 / 30 | 53/31 | 69 / 22 | 57 / 25 | 76 / 14 | 72 / 10 |
| Hb, (g/dl) | 9,8 | 10,6 | 11,7 | 11,6 | 12,8 | 15,3 | 11,8 | 9,9 | 11,7 |
| CRP (mg/l) | 0,06 | 0,11 | 0,42 | 0,05 | 0,38 | 0,21 | 0,16 | 0,08 | 0,63 |
| Tacrolimus levels (ng/ml) | 6,9 | 9 | 5,9 | 5,2 | 4,6 | 6,9 | 5,9 | 11,2 | 7,2 |
| DSA (MFI) | / | / | / | DQ7 7235 | DQ8 13600, DR53 23'000 | DQ4 4000 | Neg | Neg | Neg |

| **Urinary Characteristics** | | | | | | | | | |
|---|---|---|---|---|---|---|---|---|---|
| Proteinuria (g/24h) | 0,05 | 0,1 | 0,1 | 0,8 | 0,27 | 1,9 | 0,06 | 0,39 | 0,9 |
| Urinary Sediment | Neg | Low MH | Neg | Neg | Low MH | Neg | Neg | Low MH | Neg |
| Urine Culture | Neg | Neg | Neg | Neg | Neg | Neg | Neg | Neg | Neg |

| **Immunosuppressive therapy** | | | | | | | | | |
|---|---|---|---|---|---|---|---|---|---|
| Tacrolimus (mg/die) | 2 + 1 | 4 + 4 | 4 + 5 | 2 + 2 | 1,5 + 1 | 2 + 1 | 5 | 7 | 2,5 |
| Mycophenolate (mg/die) | 360 x 2 | 1000 x 2 | / | 500 x 2 | 360 x 2 | 500 x 2 | 1000 + 500 | 500 x 2 | 720 + 360 |
| Prednisone (mg/die) | 5 | 5 | 5 | 5 | 5 | 5 | 5 | 10 | 5 |

**Table 2. Characteristics of heart-transplanted patients. Abbreviations. CAV, chronic allograft vasculopathy; M, male; F, Female; ATG, thymoglobulin.**

| ***Diagnosis*** | CAV | | No-CAV | |
|---|---|---|---|---|
| ***General Characteristics*** | | | | |
| Age (years) | 58 | 47 | 50 | 42 |
| Date of Transplantation | 2010 | 2013 | 2013 | 2013 |
| Sex (M / F) | M | M | M | F |
| Pre-transplant disease | non-ischem ic | non-ischem ic | ischemic | non-ischem ic |

| ***Post-transplant prognosis*** | | | | |
|---|---|---|---|---|
| End stage heart failure etiology | Dilated cardiomyopath y | Dilated cardiomyopath y | Ischemic cardiomyopath y | Dilated cardiomyopath y |
| Acute rejection graft episodes (Yes / No) | Yes | Yes | Yes | No |
| ***ATG induction*** (Yes / No) | Yes | Yes | Yes | Yes |

| ***Immunosuppressive therapy*** | | | | |
|---|---|---|---|---|
| Methylprednisolone (Yes / No) | Yes | Yes | Yes | Yes |
| Mycophenolate (Yes / No) | No | Yes | Yes | Yes |
| Prednisone (Yes / No) | Yes | Yes | Yes | Yes |
| Cyclosporine (Yes / No) | Yes | Yes | Yes | Yes |
| Imuran (Yes / No) | Yes | No | No | No |

### References

1. Ast J, Arvaniti A, Fine NHF, Nasteska D, Ashford FB, Stamataki Z, et al. Super-resolution microscopy compatible fluorescent probes reveal endogenous glucagon-like peptide-1 receptor distribution and dynamics. Nat Commun. 2020;11(1):467.
2. Burmeister MA, Brown JD, Ayala JE, Stoffers DA, Sandoval DA, Seeley RJ, et al. The glucagon-like peptide-1 receptor in the ventromedial hypothalamus reduces short-term food intake in male mice by regulating nutrient sensor activity. Am J Physiol Endocrinol Metab. 2017;313(6):E651-E62.
3. Broide E, Bloch O, Ben-Yehudah G, Cantrell D, Shirin H, and Rapoport MJ. Reduced GLP-1R expression in gastric glands of patients with type 2 diabetes mellitus. J Clin Endocrinol Metab. 2014;99(9):E1691-5.
4. Fiorentino TV, Casiraghi F, Davalli AM, Finzi G, La Rosa S, Higgins PB, et al. Exenatide regulates pancreatic islet integrity and insulin sensitivity in the nonhuman primate baboon Papio hamadryas. JCI Insight. 2019;4(20).
5. Fiorentino TV, Owston M, Abrahamian G, La Rosa S, Marando A, Perego C, et al. Chronic continuous exenatide infusion does not cause pancreatic inflammation and ductal hyperplasia in non-human primates. Am J Pathol. 2015;185(1):139-50.
6. Drucker DJ, Habener JF, and Holst JJ. Discovery, characterization, and clinical development of the glucagon-like peptides. J Clin Invest. 2017;127(12):4217-27.
7. Thorens B. Expression cloning of the pancreatic beta cell receptor for the gluco-incretin hormone glucagon-like peptide 1. Proc Natl Acad Sci U S A. 1992;89(18):8641-5.
8. Andreozzi F, Raciti GA, Nigro C, Mannino GC, Procopio T, Davalli AM, et al. The GLP-1 receptor agonists exenatide and liraglutide activate Glucose transport by an AMPK-dependent mechanism. J Transl Med. 2016;14(1):229.
9. Muskiet MHA, Tonneijck L, Smits MM, van Baar MJB, Kramer MHH, Hoorn EJ, et al. GLP-1 and the kidney: from physiology to pharmacology and outcomes in diabetes. Nat Rev Nephrol. 2017;13(10):605-28.
10. Drucker DJ, and Rosen CF. Glucagon-like peptide-1 (GLP-1) receptor agonists, obesity and psoriasis: diabetes meets dermatology. Diabetologia. 2011;54(11):2741-4.
11. Hadjiyanni I, Siminovitch KA, Danska JS, and Drucker DJ. Glucagon-like peptide-1 receptor signalling selectively regulates murine lymphocyte proliferation and maintenance of peripheral regulatory T cells. Diabetologia. 2010;53(4):730-40.
12. Drucker DJ. Mechanisms of Action and Therapeutic Application of Glucagon-like Peptide-1. Cell Metab. 2018;27(4):740-56.
13. He S, Kahles F, Rattik S, Nairz M, McAlpine CS, Anzai A, et al. Gut intraepithelial T cells calibrate metabolism and accelerate cardiovascular disease. Nature. 2019;566(7742):115-9.
14. Buysschaert M, Tennstedt D, and Preumont V. Improvement of psoriasis during exenatide treatment in a patient with diabetes. Diabetes Metab. 2012;38(1):86-8.
15. Hadjiyanni I, Baggio LL, Poussier P, and Drucker DJ. Exendin-4 modulates diabetes onset in nonobese diabetic mice. Endocrinology. 2008;149(3):1338-49.
16. Xue S, Wasserfall CH, Parker M, Brusko TM, McGrail S, McGrail K, et al. Exendin-4 therapy in NOD mice with new-onset diabetes increases regulatory T cell frequency. Ann N Y Acad Sci. 2008;1150:152-6.
17. Moschovaki Filippidou F, Kirsch AH, Thelen M, Ketszeri M, Artinger K, Aringer I, et al. Glucagon-Like Peptide-1 Receptor Agonism Improves Nephrotoxic Serum Nephritis by Inhibiting T-Cell Proliferation. Am J Pathol. 2020;190(2):400-11.
18. Kubli SP, Berger T, Araujo DV, Siu LL, and Mak TW. Beyond immune checkpoint blockade: emerging immunological strategies. Nat Rev Drug Discov. 2021;20(12):899-919.
19. Seruga B, Ocana A, Amir E, and Tannock IF. Failures in Phase III: Causes and Consequences. Clin Cancer Res. 2015;21(20):4552-60.
20. Wu VH, Yung BS, Faraji F, Saddawi-Konefka R, Wang Z, Wenzel AT, et al. The GPCR-Galpha(s)-PKA signaling axis promotes T cell dysfunction and cancer immunotherapy failure. Nat Immunol. 2023;24(8):1318-30.
21. Graziano MP, Hey PJ, Borkowski D, Chicchi GG, and Strader CD. Cloning and functional expression of a human glucagon-like peptide-1 receptor. Biochem Biophys Res Commun. 1993;196(1):141-6.
22. Thorens B, Porret A, Buhler L, Deng SP, Morel P, and Widmann C. Cloning and functional expression of the human islet GLP-1 receptor. Demonstration that exendin-4 is an agonist and exendin-(9-39) an antagonist of the receptor. Diabetes. 1993;42(11):1678-82.
23. Song G, Yang D, Wang Y, de Graaf C, Zhou Q, Jiang S, et al. Human GLP-1 receptor transmembrane domain structure in complex with allosteric modulators. Nature. 2017;546(7657):312-5.
24. Montrose-Rafizadeh C, Yang H, Rodgers BD, Beday A, Pritchette LA, and Eng J. High potency antagonists of the pancreatic glucagon-like peptide-1 receptor. J Biol Chem. 1997;272(34):21201-6.
25. Nance KD, Days EL, Weaver CD, Coldren A, Farmer TD, Cho HP, et al. Discovery of a Novel Series of Orally Bioavailable and CNS Penetrant Glucagon-like Peptide-1 Receptor (GLP-1R) Noncompetitive Antagonists Based on a 1,3-Disubstituted-7-aryl-5,5-bis(trifluoromethyl)-5,8-dihydropyrimido[4,5-d]pyrimidine-2,4(1H,3H)-dione Core. J Med Chem. 2017;60(4):1611-6.
26. Peterson SM, Juliana CA, Hu CF, Chai J, Holliday C, Chan KY, et al. Optimization of a Glucagon-Like Peptide 1 Receptor Antagonist Antibody for Treatment of Hyperinsulinism. Diabetes. 2023;72(9):1320-9.
27. De Leon DD, Li C, Delson MI, Matschinsky FM, Stanley CA, and Stoffers DA. Exendin-(9-39) corrects fasting hypoglycemia in SUR-1 -/- mice by lowering cAMP in pancreatic beta-cells and inhibiting insulin secretion. J Biol Chem. 2008;283(38):25786-93.
28. Wang S, Wang Y, Lin L, Li Z, Liu F, Zhu L, et al. Layer-Specific BTX-A Delivery to the Gastric Muscularis Achieves Effective Weight Control and Metabolic Improvement. Adv Sci (Weinh). 2023;10(28):e2300822.
29. Knudsen LB, and Pridal L. Glucagon-like peptide-1-(9-36) amide is a major metabolite of glucagon-like peptide-1-(7-36) amide after in vivo administration to dogs, and it acts as an antagonist on the pancreatic receptor. Eur J Pharmacol. 1996;318(2-3):429-35.
30. Usuelli V, Ben Nasr M, D'Addio F, Liu K, Vergani A, El Essawy B, et al. miR-21 antagonism reprograms macrophage metabolism and abrogates chronic allograft vasculopathy. Am J Transplant. 2021;21(10):3280-95.
31. D'Addio F, Vergani A, Potena L, Maestroni A, Usuelli V, Ben Nasr M, et al. P2X7R mutation disrupts the NLRP3-mediated Th program and predicts poor cardiac allograft outcomes. J Clin Invest. 2018;128(8):3490-503.
32. Vergani A, Tezza S, D'Addio F, Fotino C, Liu K, Niewczas M, et al. Long-term heart transplant survival by targeting the ionotropic purinergic receptor P2X7. Circulation. 2013;127(4):463-75.
33. Ben Nasr M, Vergani A, Avruch J, Liu L, Kefaloyianni E, D'Addio F, et al. Co-transplantation of autologous MSCs delays islet allograft rejection and generates a local immunoprivileged site. Acta Diabetol. 2015;52(5):917-27.
34. Tezza S, Ben Nasr M, D'Addio F, Vergani A, Usuelli V, Falzoni S, et al. Islet-Derived eATP Fuels Autoreactive CD8(+) T Cells and Facilitates the Onset of Type 1 Diabetes. Diabetes. 2018;67(10):2038-53.
35. Vergani A, Gatti F, Lee KM, D'Addio F, Tezza S, Chin M, et al. TIM4 Regulates the Anti-Islet Th2 Alloimmune Response. Cell Transplant. 2015;24(8):1599-614.
36. Tacconi C, Correale C, Gandelli A, Spinelli A, Dejana E, D'Alessio S, et al. Vascular endothelial growth factor C disrupts the endothelial lymphatic barrier to promote colorectal cancer invasion. Gastroenterology. 2015;148(7):1438-51 e8.
37. Tacconi C, Ungaro F, Correale C, Arena V, Massimino L, Detmar M, et al. Activation of the VEGFC/VEGFR3 Pathway Induces Tumor Immune Escape in Colorectal Cancer. Cancer Res. 2019;79(16):4196-210.
38. Fotino C, Molano RD, Ben Nasr M, Umland O, Fraker CA, Ulissi U, et al. Reversal of Experimental Autoimmune Diabetes With an sCD39/Anti-CD3 Treatment. Diabetes. 2023;72(11):1641-51.
39. Petrelli A, Carvello M, Vergani A, Lee KM, Tezza S, Du M, et al. IL-21 is an antitolerogenic cytokine of the late-phase alloimmune response. Diabetes. 2011;60(12):3223-34.
40. D'Addio F, La Rosa S, Maestroni A, Jung P, Orsenigo E, Ben Nasr M, et al. Circulating IGF-I and IGFBP3 Levels Control Human Colonic Stem Cell Function and Are Disrupted in Diabetic Enteropathy. Cell Stem Cell. 2015;17(4):486-98.
41. Chesnokova LS, and Yurochko AD. Using a Phosphoproteomic Screen to Profile Early Changes During HCMV Infection of Human Monocytes. Methods Mol Biol. 2021;2244:233-46.
42. Ben Nasr M, Robbins D, Parone P, Usuelli V, Tacke R, Seelam AJ, et al. Pharmacologically Enhanced Regulatory Hematopoietic Stem Cells Revert Experimental Autoimmune Diabetes and Mitigate Other Autoimmune Disorders. J Immunol. 2022;208(7):1554-65.
43. Pasquale V, Ducci G, Campioni G, Ventrici A, Assalini C, Busti S, et al. Profiling and Targeting of Energy and Redox Metabolism in Grade 2 Bladder Cancer Cells with Different Invasiveness Properties. Cells. 2020;9(12).
44. Aran D, Looney AP, Liu L, Wu E, Fong V, Hsu A, et al. Reference-based analysis of lung single-cell sequencing reveals a transitional profibrotic macrophage. Nat Immunol. 2019;20(2):163-72.
45. Bendotti G, Montefusco L, Lunati ME, Usuelli V, Pastore I, Lazzaroni E, et al. The anti-inflammatory and immunological properties of GLP-1 Receptor Agonists. Pharmacol Res. 2022;182:106320.
46. Wang V, Kuo TT, Huang EY, Ma KH, Chou YC, Fu ZY, et al. Sustained Release GLP-1 Agonist PT320 Delays Disease Progression in a Mouse Model of Parkinson's Disease. ACS Pharmacol Transl Sci. 2021;4(2):858-69.
47. Rakipovski G, Rolin B, Nohr J, Klewe I, Frederiksen KS, Augustin R, et al. The GLP-1 Analogs Liraglutide and Semaglutide Reduce Atherosclerosis in ApoE(-/- ) and LDLr(-/-) Mice by a Mechanism That Includes Inflammatory Pathways. JACC Basic Transl Sci. 2018;3(6):844-57.
48. Tate M, Robinson E, Green BD, McDermott BJ, and Grieve DJ. Exendin-4 attenuates adverse cardiac remodelling in streptozocin-induced diabetes via specific actions on infiltrating macrophages. Basic Res Cardiol. 2016;111(1):1.
49. Tsai S, Winer S, and Winer DA. Gut T Cells Feast on GLP-1 to Modulate Cardiometabolic Disease. Cell Metab. 2019;29(4):787-9.
50. Hogan AE, Tobin AM, Ahern T, Corrigan MA, Gaoatswe G, Jackson R, et al. Glucagon-like peptide-1 (GLP-1) and the regulation of human invariant natural killer T cells: lessons from obesity, diabetes and psoriasis. Diabetologia. 2011;54(11):2745-54.
51. Buysschaert M, Baeck M, Preumont V, Marot L, Hendrickx E, Van Belle A, et al. Improvement of psoriasis during glucagon-like peptide-1 analogue therapy in type 2 diabetes is associated with decreasing dermal gammadelta T-cell number: a prospective case-series study. Br J Dermatol. 2014;171(1):155-61.
52. Wong CK, Yusta B, Koehler JA, Baggio LL, McLean BA, Matthews D, et al. Divergent roles for the gut intraepithelial lymphocyte GLP-1R in control of metabolism, microbiota, and T cell-induced inflammation. Cell Metab. 2022;34(10):1514-31 e7.
53. Geginat J, Lanzavecchia A, and Sallusto F. Proliferation and differentiation potential of human CD8+ memory T-cell subsets in response to antigen or homeostatic cytokines. Blood. 2003;101(11):4260-6.
54. Doan Ngoc TM, Tilly G, Danger R, Bonizec O, Masset C, Guerif P, et al. Effector Memory-Expressing CD45RA (TEMRA) CD8(+) T Cells from Kidney Transplant Recipients Exhibit Enhanced Purinergic P2X4 Receptor-Dependent Proinflammatory and Migratory Responses. J Am Soc Nephrol. 2022;33(12):2211-31.

## Claims

1. A GLP-1R antagonist or inhibitor for use for the prevention and/or treatment of a cancer.

2. The GLP-1R antagonist for the use according to claim 1, wherein said GLP-1R antagonist is selected from the group consisting of Exendin fragment (9-39), Exendin-3 (9-39) amide, GLP-1R Antagonist 1 (compound 5d), TB-001-003, TB-222-023 or Avexitide, VU 0650991, GLP-1 (9-36) amide, a molecule able to bind to GLP-1R⁺ cells and CD3⁺ T cells and a monoclonal antibody against GLP-1R, or functional fragments or derivatives thereof.

3. The GLP-1R antagonist or inhibitor for the use according to claim 1 or 2 wherein said tumor is a solid tumor selected from the group consisting of colorectal cancer, gastrointestinal cancer, pancreatic cancer, lung cancer, breast cancer, bladder cancer, prostate cancer, testicular cancer, ovarian cancer, uterine cancer, stomach cancer, liver cancer, renal cancer, melanoma, retinoblastoma, neuroblastoma, and sarcoma, or a liquid tumor selected from the group consisting of leukemias, lymphoma, and multiple myeloma.

4. The GLP-1R antagonist or inhibitor for the use according to any one of the previous claims wherein said GLP-1R antagonist or inhibitor is for use for the prevention and/or treatment of a cancer in a subject who is or has been treated with an immune checkpoint inhibitor, such as an antibody anti-PD-1, anti-PDL-1 or anti-CTLA-4.

5. The GLP-1R antagonist or inhibitor for the use according to any one of the previous claims, wherein the GLP-1R antagonist or inhibitor is administered before, simultaneously or after a further cancer treatment, such as chemotherapy, radiotherapy, immunotherapy or combinations thereof.

6. The GLP-1R inhibitor for the use according to any one of the previous claims wherein said inhibitor is a nucleic acid molecule selected from: a single-stranded or double-stranded nucleic acid molecule, a miRNA molecule, a siRNA molecule, a shRNA molecule, an antisense oligonucleotide, such as an antagomir or a Locked Nucleic Acid (LNA) modified ASO (LNA-ASO), derivatives and mixtures thereof or it is a vector, preferably a recombinant expression vector, comprising a coding sequence for said inhibitor and/or expressing said inhibitor, preferably under the control of a suitable promoter.

7. The GLP-1R inhibitor for the use according to any one of the previous claims, wherein said inhibitor is a vector targeting GLP-1R gene to be administered together with a vector expressing a nuclease in order to knockdown GLP-1R using a CRISPR/Cas9 system.

8. A GLP-1R antagonist or inhibitor for use for improving the effect of a cancer treatment, said cancer treatment being preferably chemotherapy, radiotherapy, immunotherapy or combinations thereof, wherein preferably said GLP-1R antagonist or inhibitor is as described in any of claims 2, 6 or 7.

9. An isolated polynucleotide comprising at least one sequence that encodes a GLP-1R antagonist or inhibitor, preferably a GLP-1R antagonist or inhibitor as described in any of claims 2, 6 or 7, for use in the prevention and/or treatment of a cancer.

10. A vector comprising or expressing the polynucleotide according to claim 9, preferably said vector being selected from the group consisting of a plasmid, a viral vector, a non-episomal mammalian vector, an expression vector and a recombinant expression vector, for use in the prevention and/or treatment of a cancer.

11. A pharmaceutical composition comprising a GLP-1R antagonist or inhibitor and at least one pharmaceutically acceptable carrier, excipient and/or stabilizer for use in the prevention and/or treatment of a cancer or for use for improving the effect of a cancer treatment.

12. The pharmaceutical composition for the use according to claim 11 which further comprises a further therapeutic agent.

13. The GLP-1R antagonist or inhibitor for the use according to any one of claims 1-8 wherein said GLP-1R antagonist or inhibitor is used in combination with at least one further therapeutic agent.

14. The GLP-1R antagonist or inhibitor for use according to claim 13 or the pharmaceutical composition for use according to claim 12 wherein said further therapeutic agent is an active ingredient which is known to be effective in the prevention and/or treatment of a cancer, such as a IDO/TDO inhibitor, such as Epacadostat, an anti-EGFR such as cetuximab, a platinum-based chemotherapy, e.g. cisplatin, carboplatin, or oxaliplatin, Paclitaxel/Carboplatin, a MEK inhibitor such as cobimetinib or regorafenib, a ERBB2- targeted antibody drug conjugate such as trastuzumab emtansine, Ipilimumab (BMS-734016), an anti-CTLA-4 such as Yervoy or Tremelimumab, an anti-PD-L1 such as durvalumab or atezolizumab(anti-PD-L1), or an anti-PD-1 such as nivolumab.
